# EUROPÄISCHE PATENTSCHRIFT

(11) **EP 1 513 811 B1**
(45) Veröffentlichungstag und Bekanntmachung des Hinweises auf die Patenterteilung: **08.04.2009**
(21) Anmeldenummer: 03757007.4
(22) Anmeldetag: 30.05.2003
(51) Int. Cl.: C07D 209/20, C07K 5/087, A61K 31/405, A61K 38/05, A61K 38/06, A61P 3/04, A61P 3/10, A61P 9/00, C07K 5/062, C07K 5/078, C07K 5/072, C07D 401/12, C07D 405/12

(54) **NICHT-PEPTIDISCHE BRS-3-AGONISTEN**
NON-PEPTIDIC BRS-3 AGONISTS
AGONISTES DU RECEPTEUR BRS-3 NON PEPTIDIQUES

(30) Priorität: 05.06.2002 DE 10224844
(43) Veröffentlichungstag der Anmeldung: 16.03.2005
(73) Patentinhaber: Solvay Pharmaceuticals GmbH, 30173 Hannover (DE)
(72) Erfinder: WEBER, Dirk, 73235 Weilheim (DE); KESSLER, Horst, 85748 Garching (DE); BERGER, Claudia, 71522 Backnang (DE); ANTEL, Jochen, 31848 Bad Münder (DE); HEINRICH, Timo, 64823 Gross-Umstadt (DE)
(74) Vertreter: Bauriegel, Lutz
(86) Internationale Anmeldenummer: PCT/EP2003/005678
(87) Internationale Veröffentlichungsnummer: WO 2003/104196

(56) Entgegenhaltungen:
- WO-A-96/31214
- WO-A-97/06803
- MANTEY SAMUEL A ET AL: "Rational design of a peptide agonist that interacts selectively with the orphan receptor, bombesin receptor subtype 3" JOURNAL OF BIOLOGICAL CHEMISTRY, THE AMERICAN SOCIETY OF BIOLOGICAL CHEMISTS, INC.,, US, Bd. 276, Nr. 12, 23. März 2001 (2001-03-23), Seiten 9219-9229, XP002186408 ISSN: 0021-9258 in der Anmeldung erwähnt
- WEBER D ET AL.: "Systematic optimization of a lead-structure identities for a selective short peptide agonist for the human orphan receptor BRS-3" JOURNAL OF PEPTIDE SCIENCE., Bd. 8, Nr. 8, August 2002 (2002-08), Seiten 461-475, XP009015889 JOHN WILEY AND SONS LTD., GB ISSN: 1075-2617
- WEBER D ET AL.: "Design of selective peptidomimetic agonists for the human orphan receptor BRS-3" JOURNAL OF MEDICINAL CHEMISTRY., Bd. 46, Nr. 10, 2003, Seiten 1918-1930, XP002251681 AMERICAN CHEMICAL SOCIETY. WASHINGTON., US ISSN: 0022-2623

## Beschreibung

Die vorliegende Erfindung betrifft neue, nicht-peptidische Verbindungen, die eine selektive agonistische Wirkung auf den Bombesin-Rezeptor des Subtyps 3 (BRS-3) zeigen, und diese Verbindungen enthaltende pharmazeutische Zubereitungen sowie Verfahren zur Herstellung dieser Verbindungen.

Bombesin (Bn) ist ein aus 14 Aminosäuren bestehendes Peptid, welches ursprünglich aus Amphibien isoliert wurde. Die beiden in Säugetieren identifizierten Peptide Neuromedin B (NMB) und das "Gastrin-Releasing Peptide" (GRP) stellen strukturell ähnliche Peptide dar. Diese Bombesin-ähnlichen Peptide sind die natürlichen endogenen Liganden der entsprechenden Bombesin-Rezeptoren, des "Neuromedin B-Rezeptors" (NMB-R, BB1) und des "Gastrin-Releasing Peptide-Rezeptors" (GRP-R, BB2). Die Bombesin-Rezeptbren zählen zur Gruppe der G-gekoppelten Rezeptoren mit 7 Transmembran-Domänen.

Der Bombesin Rezeptor des Subtyps 3 (BRS-3 oder BB3) wird aufgrund der Homologie seiner Aminosäuresequenz dieser Familie der Bombesin-Rezeptoren zugeordnet [vgl. Fathi et al. (1993) J Biol Chem. 268:5979-84; nachfolgend zitiert als "Fathi *et al*."]. Der natürliche Ligand von BRS-3 ist bisher nicht bekannt. Die Expression von BRS-3 konnte in verschiedenen Gehirnregionen [vgl. Yamada et al. (1999) Physiol Behav. 66:863-7], in sekundären Spermatozyten [vgl. Fathi *et al*.], in pankreatischen Inselzellen [vgl. Fleischmann et al. (2000) Lab Invest. 80:1807-17] sowie im Uterusgewebe trächtiger Tiere [vgl. Gorbulev et al. (1992) Eur J Biochem. 208:405-10] nachgewiesen werden. Außerdem wurde BRS-3 auf unterschiedlichen humanen Krebszell-Linien (z.B. Lunge [vgl. Fathi et *al*.], Brust [vgl. Gorbulev et al. (1994) FEBS Lett. 340:260-4], Prostata [vgl. Sun et al. (2000) Prostate. 42:295-303] oder Ovarien [vgl. Sun et al. (2000) Regul Pept. 90:77-84]) identifiziert.

Genetisch veränderte Mäuse, in denen das BRS-3 Gen ausgeschaltet wurde ("BRS-3 Knockout Mice"), zeigten ein Krankheitsbild, welches Fettleibigkeit (Obesitas), Hyperphagie sowie Bluthochdruck und Diabetes umfasste [vgl. Okhi-Hamazaki et al. (1997) Nature 390:165-9]. Demnach scheint BRS-3 an der Regulation des Glucose- und des Fettstoffwechsels, am Erhalt des Energiestatus und an der Kontrolle des Blutdrucks, sowie an der Beeinflussung des Essverhaltens essentiell beteiligt zu sein. Von BRS-3-agonistisch wirksamen Verbindungen kann daher angenommen werden, daß sie insbesondere zur Prophylaxe und/oder Behandlung krankhafter Zustände wie Fettleibigkeit (= Obesitas, Adipositas), Diabetes, Hyperinsulinämie, kardiovaskulären Erkrankungen, Essstörungen (Hyperphagie, Anorexie, Bulimie) und/oder dem metabolischen Syndrom (= Syndrom X) geeignet sind. Syndrom X manifestiert sich vor allem durch Diabetes mellitus Typ II bzw. eine verminderte Glucosetoleranz, arterielle Hypertonie, Störungen des Fettstoffwechsels, Adipositas sowie durch die koronare Herzkrankheit.

Weiterhin ist bekannt, daß die Aktivierung von BRS-3 eine neuroprotektive Wirkung haben kann [vgl. WO 01/68120]. Auch scheint BRS-3 mit der Geschmackswahrnehmung [vgl. Yamada et al. (1999) Physiol Behav. 66:863-7], der Beeinflussung des Sozialverhaltens,[vgl. Yamada et al. (2000) Physiol Behav. 68:555-61] und bestimmter emotionaler Verhaltensweisen [vgl. Yamada et al. (2002) Mol Psychiatry. 7:113-7] im Zusammenhang zu stehen. Es kann daher ebenfalls angenommen werden, dass BRS-3-modulatorisch wirksame Verbindungen zur Prophylaxe und/oder Behandlung psychischer Krankheitsbilder wie Depression oder Angstzuständen, Störungen der Geschmackswahrnehmung und/oder degenerativer Erkrankungen des zentralen Nervensystems, beispielsweise Parkinson oder Alzheimer, geeignet sein können.

Es sind bereits einige synthetische peptidische Liganden bekannt, die mit gewisser Affinität an BRS-3 binden und dort eine agonistische Wirkung entfalten, nämlich das BRS-3 selektive Oktapeptid [D-Phe⁶, Phe¹³]Bn(6-13) Propylamid [vgl. Wu et al. (1996) Mol Pharmacol. 50:1355-63] sowie das weniger selektive Nonapeptid [D-Tyr⁶, β-Alall, Phe¹³, Nle¹⁴]Bn(6-14) [vgl. Mantey et al. (1997) J Biol Chem. 272:26062-71] und dessen Derivate [vgl. Pradhan et al. (1998) Eur J Pharmacol. 343:275-87; Mantey et al. (2001) J Biol Chem. 276:9219-29].

Aus der WO 98/07718 sind weiterhin bereits niedermolekulare, nicht-peptidische Bombesin-analoge Verbindungen bekannt, welche aber selektive Antagonisten der beiden anderen Subtypen der Bombesin-Rezeptor-Familie (NMB-R und GRP-R) darstellen. Niedermolekulare, nicht-peptidische und selektiv mit hoher Affinität am BRS-3 agonistisch wirksame Verbindungen sind dagegen bislang nicht beschrieben worden.

Der vorliegenden Erfindung liegt daher die Aufgabe zu Grunde, neue niedermolekulare, nicht-peptidische und selektiv mit hoher Affinität am BRS-3 agonistisch wirksame Verbindungen zur Verfügung zu stellen.

Es wurde nun überraschend gefunden, daß die erfindungsgemäßen niedermolekularen und nicht-peptidischen neuen Verbindungen selektive BRS-3-Agonisten sind und sich somit zur Prophylaxe und/oder Behandlung von Krankheitsbildern eignen, welche durch eine Stimulierung des BRS-3 günstig beeinflußt werden können. Aufgrund ihres Wirkprofiles erscheinen die erfindungsgemäßen Verbindungen insbesondere geeignet zur Prophylaxe und/oder Behandlung von Fettleibigkeit (= Obesitas, Adipositas), Diabetes, Hyperinsulinämie, kardiovaskulären Erkrankungen, Essstörungen (Hyperphagie, Anorexie, Bulimie) und/oder Syndrom X.

Gegenstand der Erfindung sind neue Verbindungen der allgemeinen Formel I, 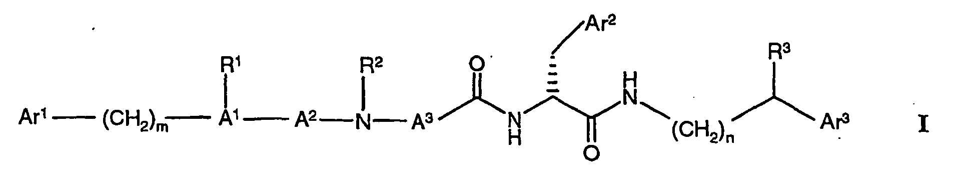 worin
- A¹: CH oder, sofern nicht A² für eine Bindung und gleichzeitig A³ für NH steht, auch Stickstoff bedeutet,
- A²: eine Bindung, C₁₋₂-Alkylen oder, sofern A¹ für CH steht und R² für Wasserstoff steht, auch Carbonyl bedeutet,
- A³: Methylen, welches gegebenenfalls durch C₁₋₄-Alkyl oder C₁₋₄-Alkylcarbonylamid substituiert ist, oder, sofern R² Wasserstoff bedeutet oder gemeinsam mit R¹ für eine Bindung steht, auch NH bedeutet,
- R¹: Wasserstoff oder, sofern A² für Carbonyl steht, auch Amino bedeutet und
- R²: Wasserstoff bedeutet, oder
- R¹ und R²: gemeinsam für eine Bindung stehen , sofern A² eine Bindung bedeutet
- R³: Wasserstoff oder Methyl bedeutet,
- Ar¹: Phenyl, welches gegebenenfalls 1- bis 2-fach durch Halogen oder C₁₋₄-Alkyl oder durch an zwei benachbarte Ringkohlenstoffatome gebundenes C₁₋₂-Alkylendioxy substituiert ist, Pyridyl, Furyl, Indolyl oder Tetrahydroisochinolyl bedeutet,
- Ar²: Furyl, Benzofuranyl, Thienyl, Benzothiophenyl, Pyrrolyl oder Indolyl bedeutet,
- Ar³: Phenyl, welches gegebenenfalls 1 bis 2-fach durch Halogen substituiert ist, oder Pyridyl bedeutet,
- m: 0 oder 1 bedeutet und
- n: 0 oder 1 bedeutet
sowie gegebenenfalls deren physiologisch verträgliche Säureadditionssalz. Ferner sind Gegenstand der Erfindung die Verbindungen der Formel I enthaltende pharmazeutische Zubereitungen sowie ein Verfahren zur Herstellung dieser Verbindungen.

Sofern in den Verbindungen der Formel I Substituenten C₁₋₄-Alkyl enthalten sind, kann dieses geradkettig oder verzweigt sein. Sofern Substituenten Halogen enthalten, kann dieses insbesondere Fluor, Chlor oder Brom bedeuten. Chlor ist bevorzugt.

Sofern A³ durch C₁₋₄-Alkyl substituiert ist, ist Methyl bevorzugt. Sofern A³ durch C₁₋₄-Alkylcarbonylamid substituiert ist, ist n-Propylamid bevorzugt.

R³ steht vorzugsweise für Wasserstoff.

Ar¹ bedeutet vorzugsweise Phenyl, welches gegebenenfalls 1-fach durch Halogen substituiert ist, Pyridyl, Furyl, insbesondere 2-Furyl, oder Indolyl, insbesondere 2-Indolyl.

Ar² steht vorzugsweise für Benzothiophenyl oder für Indolyl. Indolyl, insbesondere 3-Indolyl, ist bevorzugt.

Ar³ steht vorzugsweise für Phenyl, insbesondere unsubstituiertes Phenyl.

n bedeutet vorzugsweise 1.

Bevorzugte Verbindungen der Formel I stellen die nachfolgend angegebenen Verbindungen der allgemeinen Formel Ia, 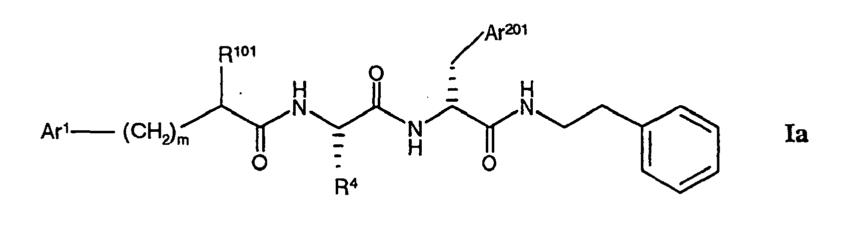 worin Ar¹ und m obige Bedeutungen besitzen, R¹⁰¹ Wasserstoff oder Amino bedeutet, R⁴ Wasserstoff, C₁₋₄-Alkyl oder C₁₋₄-Alkylcarbonylamid bedeutet und Ar²⁰¹ Benzothiophenyl oder Indolyl bedeutet, der allgemeinen Formel Ib, 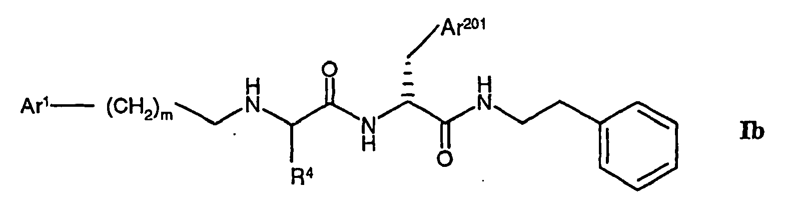 worin R⁴, Ar¹, Ar²⁰¹ und m obige Bedeutungen besitzen, der allgemeinen Formel Ic, 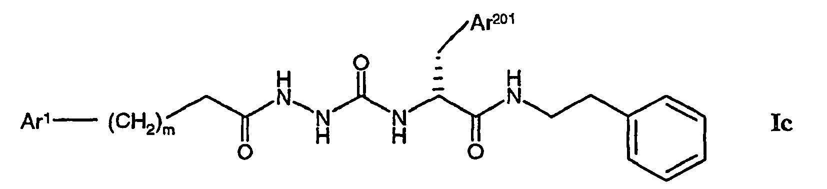 worin Ar¹; Ar²⁰¹ und m obige Bedeutungen besitzen, der allgemeinen Formel Id, 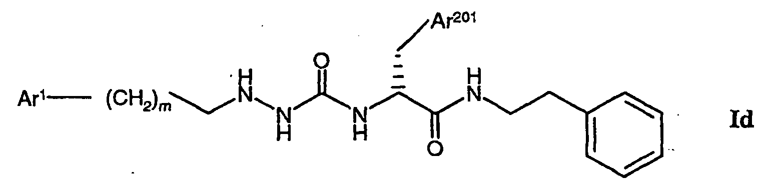 worin Ar¹, Ar²⁰¹ und m obige Bedeutungen besitzen und der allgemeinen Formel Ie, 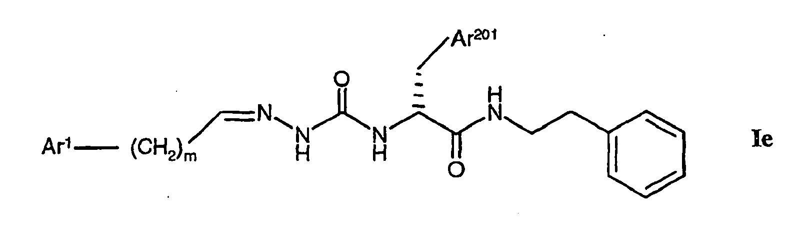 worin Ar¹, Ar²⁰¹ und m obige Bedeutungen besitzen, dar.

Die Verbindungen der Formel I stellen nicht-peptidische Verbindungen dar, welche aber Peptidbindungen enthalten. Die Verbindungen der Formel I können daher als nicht-natürliche Polypeptide aufgefaßt und teilweise oder vollständig auf an sich zur Polypeptidsynthese bekannte Weise, beispielsweise durch übliche Fest- oder Flüssigphasensynthesetechniken mit geeigneten Amino- und Carboxylbausteinen, vorzugsweise sequentiell aufgebaut werden. Sofern zusätzlich auch andere organisch-chemische Synthesemethoden zum Aufbau der Verbindungen der Formel I eingesetzt werden, können an sich bekannte klassische organisch-chemische Synthesemethoden eingesetzt werden.

So können die Verbindungen der Formel I und deren Säureadditionssalze beispielsweise hergestellt werden, indem man
a) zur Herstellung einer Verbindung der allgemeinen Formel Ig, 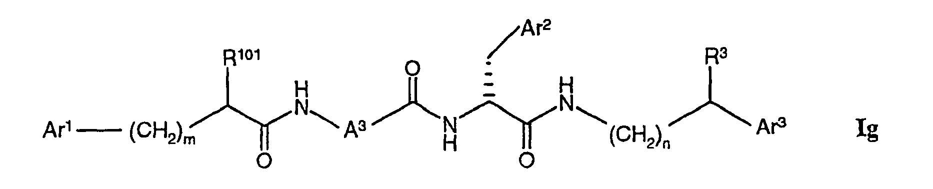 worin A³, R¹⁰¹, R³, Ar¹, Ar², Ar³, mund n obige Bedeutungen besitzen, eine Verbindung der allgemeinen Formel II, 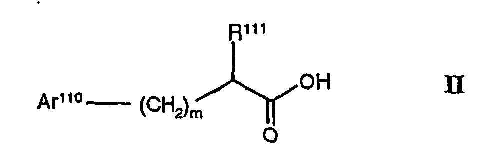 worin m obige Bedeutung besitzt, Ar¹¹⁰ die oben für Ar¹ angegebene Bedeutung besitzt, wobei allfällige reaktive Gruppen durch Schutzgruppen geschützt sind und R¹¹¹ die oben für R¹⁰¹ angegebene Bedeutung besitzt, wobei eine allfällige Aminogruppe durch eine Schutzgruppe geschützt ist, mit einer Verbindung der allgemeinen Formel III, 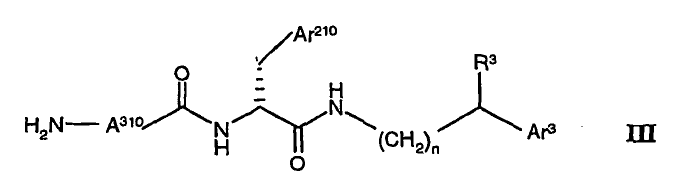 worin R³, Ar³ und n obige Bedeutungen besitzen, Ar²¹⁰ die oben für Ar² angegebene Bedeutung besitzt, wobei allfällige reaktive Gruppen durch Schutzgruppen geschützt sind und A³¹⁰ die oben für A³ angegebene Bedeutung besitzt, wobei allfällige reaktive Stickstoffatome durch Schutzgruppen geschützt sind, umsetzt, oder
b) zur Herstellung einer Verbindung der allgemeinen Formel Ih 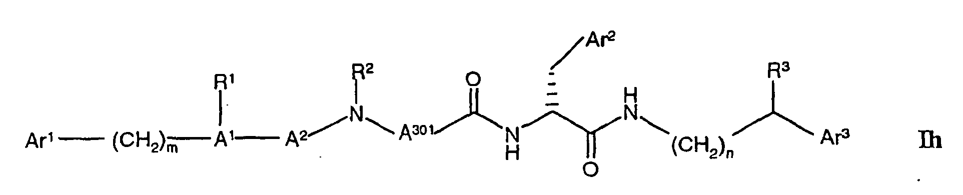 worin A¹, A² , R¹ , R² , R³ , Ar¹ , Ar² , Ar³ , m und n obige Bedeutungen besitzen und A³⁰¹ die oben für A³ angegebene Bedeutung mit Ausnahme von NH besitzt, eine Verbindung der allgemeinen Formel IV, 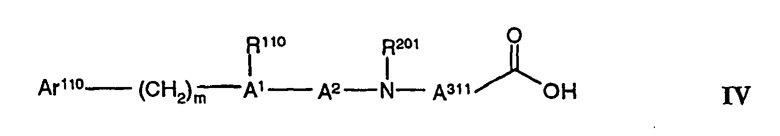 worin A¹, A², Ar¹¹⁰ und m obige Bedeutungen besitzen, A³¹¹ die oben für A³⁰¹ angegebene Bedeutung besitzt, wobei allfällige reaktive Stickstoffatome durch Schutzgruppen geschützt sind, R¹¹⁰ die oben für R¹ angegebene Bedeutung besitzt, wobei eine allfällige Aminogruppen durch eine Schutzgruppe geschützt ist und R²⁰¹ die oben für R² angegebene Bedeutung mit der Ausnahme von Wasserstoff besitzt, oder eine Aminoschutzgruppe bedeutet, mit einer Verbindung der allgemeinen Formel V, 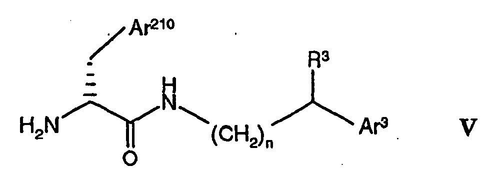 worin R³, Ar²¹⁰, Ar³ und n obige Bedeutungen besitzen, umsetzt, oder
c) zur Herstellung einer Verbindung der allgemeinen Formel Ii, 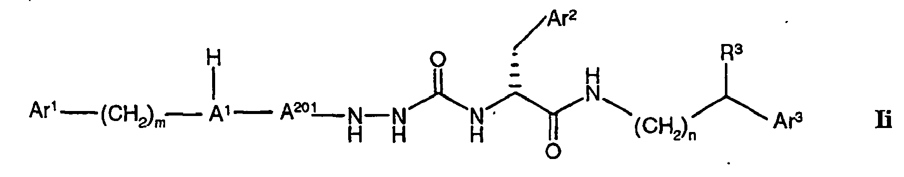 worin A¹, R³, Ar¹, Ar², Ar³, m und n obige Bedeutungen besitzen und A²⁰¹ die oben für A² angegebene Bedeutung mit Ausnahme von Carbonyl besitzt, eine Verbindung der Formel V mit einem Carbonylgruppen-Syntheseäquivalent und mit einer Verbindung der allgemeinen Formel VI, 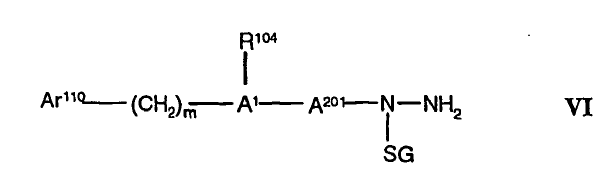 worin A¹, A²⁰¹, Ar¹¹⁰ und m obige Bedeutungen besitzen, R¹⁰⁴ für Wasserstoff oder, sofern A¹ Stickstoff bedeutet, auch für eine Stickstoffschutzgruppe stehen kann, und SG für eine in der Peptidchemie geeignete Schutzgruppe steht, umsetzt, oder
d) zur Herstellung einer Verbindung der allgemeinen Formel Ij, 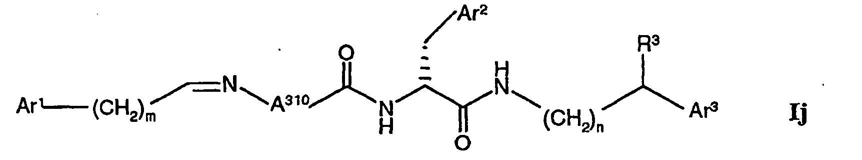
worin A³¹⁰, R³, Ar¹, Ar², Ar³, m und n obige Bedeutungen besitzen, eine Verbindung der Formel III mit einer Verbindung der allgemeinen Formel VIII, 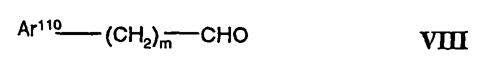 worin Ar¹¹⁰ und m obige Bedeutungen besitzen, umsetzt, und allfällige Schutzgruppen jeweils nachfolgend wieder abspaltet, und eine erhaltene Verbindung der Formel I gewünschtenfalls in ihr Säureadditionssalz überführt oder ein Säureadditionssalz in eine freie Verbindung der Formel I überführt.

Gemäß Verfahrensvariante a) kann eine Verbindung der Formel Ig hergestellt werden, indem man ein Carbonsäurederivat der Formel II mit einem primären Amin der Formel III umsetzt und gegebenenfalls vorhandene Schutzgruppen nachfolgend wieder abspaltet. Die Umsetzung kann auf in der Peptidchemie an sich bekannte Weise als Reaktion in flüssiger Phase oder auch als Festphasenreaktion, beispielsweise im Sinne einer "Merrifield"-Festphasenpeptidsynthese, durchgeführt werden. Sofern an fester Phase synthetisiert wird, wird vor zusweise eine harzgebundene Verbindung der Formel III in einem polaren aprotischen Lösungsmittel wie N-Methylpyrrolidinon (= NMP) mit einer Verbindung der Formel III sowie mit als Kopplungsreagenzien geeigneten Verbindungen, insbesondere N-Hydroxybenzotriazol (= HOBT), 2-(1H-Benzotriazol-1-yl)-1,1,3,3-tetramethyluroniumtetrafluoroborat (= TBTU), N-Hydroxy-9-azabenzotriazol (= HOAt) und/oder 2-(1*H*-9-azabenzotriazol-1-yl)-1,1,3,3-tetramethyluroniumhexafluorphosphat (= HATU) sowie in Anwesenheit einer nicht-nucleophilen organischen Base, insbesondere Diisopropylethylamin (= DIPEA) umgesetzt. Als Harz für die Festphasensynthese eignet sich insbesondere 2-(4-Formyl-3-methoxyphenoxy)ethyl-Harz (= FMPE-Harz, vgl. z.B. A. Floersheimer et al., Pept. 1990, Proc. Eur. Pept. Symp., 21th (1991), Meeting Date 1990, E. Giralt et al. (eds.) ESCOM: Leiden, 1991; 131). Die Beladung des Harzes mit der jeweils zur weiteren Umsetzung vorgesehenen Verbindung kann auf an sich bekannte Weise erfolgen (s.u.).

Verbindungen der Formel II sind an sich bekannt oder können auf an sich bekannte Weise aus bekannten Verbindungen hergestellt werden (vgl. z.B. R. Gretler et al. (1978) Helv Chim Acta 61(5):1730-1755). So können beispielsweise Verbindungen der Formel II, worin Ar¹¹⁰ gegebenenfalls geschütztes 2-Indolyl darstellt, auf an sich bekannte Weise durch reduktive Umsetzung von Nitrophenylacetessigester-Derivaten mit Titantrichlorid erhalten werden (vgl. z.B. C.J. Moody et al. (1990) J Chem Soc Perkin Trans 1:673-679; A. Mai et al. (1999) J Med Chem 42:619-627). Schutzgruppen, welche im Rahmen der vorliegenden Erfindung eingesetzt werden, können jeweils auf an sich bekannte Weise und meist selektiv und unabhängig voneinander eingeführt und wieder abgespalten werden. Geeignete Schutzgruppen für die Peptidsynthese sind beispielsweise bekannt aus J.A.W. McOmie, "Protective Groups in Organic Chemistry", Plenum Press 1971, oder T.W. Green und P.G.M. Wuts, "Protective Groups in Organic Synthesis", Wiley and Sons 1999. Sofern Substituenten R¹¹¹ durch geeignete Schutzgruppen geschützt sind, eignen sich insbesondere aus der Peptidchemie bekannte Schutzgruppen. Vorzugsweise eignen sich die tert.-Butylcarbonyloxy (= Boc)- oder die (9*H*-Fluoren-9-ylmethoxy)carbonyl (= Fmoc)-Schutzgruppe.

Verbindungen der Formel III können beispielsweise hergestellt werden, indem man eine Verbindung der Formel V mit einer Verbindung der allgemeinen Formel X, 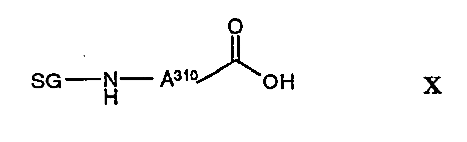 worin A³¹⁰ die obige Bedeutung besitzt und SG obige Bedeutung besitzt und vorzugsweise die Fmoc-Schutzgruppe bedeutet, umsetzt, und die Schutzgruppe SG anschließend auf an sich bekannte Weise wieder abspaltet. Die Umsetzung kann in der oben für die Umsetzung einer Verbindung der Formel II mit einer Verbindung der Formel III angegebenen Weise durchgeführt werden, wobei vorzugsweise die Verbindung der Formel V harzgebunden ist. Sofern in der Gruppe A³¹⁰ vorhandene reaktive Stickstoffatome durch geeignete Schutzgruppen geschützt sind, eignet sich hierfür insbesondere die Triphenylmethyl (= Trityl, Trt)-Schutzgruppe. Verbindungen der Formel X sind an sich bekannt oder können auf an sich bekannte Weise aus bekannten Verbindungen hergestellt werden.

Verbindungen der Formel V können hergestellt werden, indem man eine Verbindung der allgemeinen Formel XI, 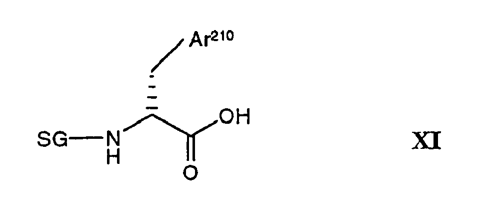 worin Ar²¹⁰ und SG die obigen Bedeutungen besitzen, mit einer Verbindung der allgemeinen Formel XII, 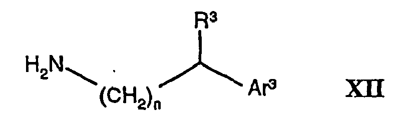 worin R³, Ar³ und n obige Bedeutungen besitzen, umsetzt, und eine Schutzgruppe SG nachfolgend wieder abspaltet. Die Umsetzung kann beispielsweise in der oben für die Umsetzung einer Verbindung der Formel II mit einer Verbindung der Formel III angegebenen Weise als Festphasensynthese durchgeführt werden, wobei vorzugsweise die Verbindung der Formel XII harzgebunden ist. Sofern FMPE-Harz eingesetzt wird, kann die Beladung.des Harzes mit einer Verbindung der Formel XII auf an sich bekannte Weise im Sinne einer reduktiven Aminierung erfolgen (vgl. B. Dörner et al., Pept. 1998, Proc. Eur. Pept. Symp, 25° (1999), Meeting Date 1998; S. Bajusz et al. (eds.), Akadémiai Kiadó: Budapest, 1999; 90). Verbindungen der Formel XI sind an sich bekannt oder können auf an sich bekannte Weise aus bekannten Verbindungen hergestellt werden. Sofern Substituenten Ar²¹⁰ in Verbindungen der Formel XI durch Schutzgruppen geschützt sind, eignen sich insbesondere aus der Peptidchemie bekannte Schutzgruppen. Vorzugsweise eignet sich die Boc-Schutzgruppe. Verbindungen der Formel XII sind an sich bekannt oder können auf an sich bekannte Weise aus bekannten Verbindungen hergestellt werden.

In einer Ausführungsform der Verfahrensvariante a) kann eine Verbindung der Formel Ik, 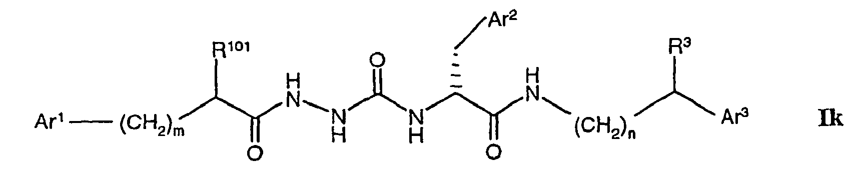 worin R¹⁰¹, R³, Ar¹, Ar², Ar³, munden obige Bedeutungen besitzen, hergestellt werden, indem man ein Carbonsäurederivat der Formel II mit einem Hydrazinderivat der Formel IIIa, 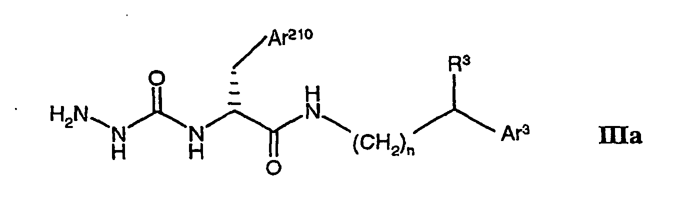 worin R³, Ar²¹⁰, Ar³ und n obige Bedeutungen besitzen, umsetzt und gegebenenfalls vorhandene Schutzgruppen nachfolgend wieder abspaltet. Die Umsetzung kann beispielsweise in der oben für die Umsetzung einer Verbindung der Formel II mit einer Verbindung der Formel III angegebenen Weise als Festphasensynthese durchgeführt werden. Verbindungen der Formel IIIa können durch Umsetzung einer Verbindung der Formel V mit an sich bekanntem 5-(9*H*-Fluoren-9-ylmethoxy)-3*H*-[1,3,4]oxadiazol-2-on und nachfolgender Abspaltung unerwünschter Schutzgruppen hergestellt werden. Die Umsetzung kann beispielsweise in der oben für die Umsetzung einer Verbindung der Formel II mit einer Verbindung der Formel III angegebenen Weise als Festphasensynthese durchgeführt werden, wobei als Lösungsmittel insbesondere Dichlormethan eingesetzt werden kann.

Gemäß Verfahrensvariante b) kann eine Verbindung der Formel Ih hergestellt werden, indem man ein Carbonsäurederivat der Formel IV mit einem primären Amin der Formel V umsetzt und gegebenenfalls vorhandene Schutzgruppen nachfolgend wieder abspaltet. Die Umsetzung kann beispielsweise in der oben für die Umsetzung einer Verbindung der Formel II mit einer Verbindung der Formel III angegebenen Weise als Festphasensynthese oder auch in flüssiger Phase durchgeführt werden. Sofern die Umsetzung an fester Phase durchgeführt wird, ist vorzugsweise die Verbindung der Formel V harzgebunden. Sofern die Umsetzung in flüssiger Phase durchgeführt wird, kann in einem polaren aprotischen Lösungsmittel wie Dimethylformamid (= DMF) und in Anwesenheit von unter Verfahrensvariante a) angegebenen, als Kopplungsreagenzien geeigneten Verbindungen sowie in Anwesenheit einer nicht-nucleophilen organischen Base, insbesondere sym. Collidin, gearbeitet werden. Sofern R²⁰¹ eine Aminoschutzgruppe bedeutet, kann dies vorzugsweise die Fmoc-Schutzgruppe sein. Sofern Substituenten R¹¹⁰ durch geeignete Schutzgruppen geschützt sind, eignen sich die oben für Substituenten R¹¹¹ als geeignet,angegebenen Schutzgruppen. Verbindungen der Formel IV können hergestellt werden, indem man eine Verbindung der allgemeinen Formel XIII, 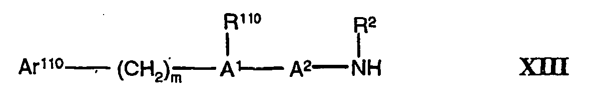 worin A¹, A², R¹¹⁰, R², Ar¹¹⁰ und m obige Bedeutungen besitzen, mit einer Verbindung der allgemeinen Formel XIV, 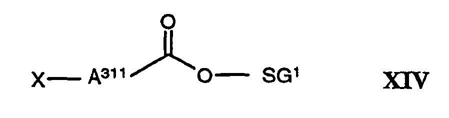 worin A³¹¹ obige Bedeutung besitzt, X für eine abspaltbare Fluchtgruppe steht und SG¹ für eine Carbonsäureschutzgruppe steht, umsetzt, eine Carbonsäureschutzgruppe SG¹ nachfolgend auf an sich bekannte Weise wieder abspaltet und nötigenfalls in Substituenten R² eine Schutzgruppe einführt. Die Umsetzung kann in einem aromatische Lösungsmittel wie Toluol bei Temperaturen zwischen -20°C und Raumtemperatur (= RT), vorzugsweise bei 0 °C, durchgeführt werden. Als Fluchtgruppe X in Verbindungen der Formel XIV kommt insbesondere Halogen, vorzugsweise Chlor oder Brom, in Frage. Als Carboxylschutzgruppe SG¹ kommt insbesondere niederes Alkyl, vorzugsweise Ethyl oder tert-Butyl in Frage. Verbindungen der Formel XIII sind an sich bekannt oder können auf an sich bekannte Weise aus bekannten Verbindungen hergestellt werden. Verbindungen der Formel XIV sind an sich bekannt oder können auf an sich bekannte Weise aus bekannten Verbindungen hergestellt werden.

Gemäß Verfahrensvariante c) kann eine Verbindung der Formel Ii hergestellt werden, indem man ein primäres Amin der Formel V mit einem Carbonylgruppen-Syntheseäquivalent und mit einem Hydrazinderivat der Formel VI umsetzt und gegebenenfalls vorhandene Schutzgruppen nachfolgend wieder abspaltet. Die Umsetzung kann vorzugsweise bei Raumtemperatur in flüssiger Phase, insbesondere in einem dipolar aprotischen Lösungsmittel wie Dichlormethan, durchgeführt werden. Zweckmäßigerweise wird in Anwesenheit einer in dem Lösungsmittel löslichen organischen nicht-nucleophilen Base wie 4-Dimethylaminopyridin (= DMAP) gearbeitet. Als Carbonylgruppen-Synthese-äquivalente eignen sich vorzugsweise Dipentafluorphenylcarbonat oder auch Phosgen, Bis-(trichlormethyl)-carbonat (Triphosgen), Chlorameisensäure-trichlormethylester (Diphosgen) oder Carbonyldiimidazol. Verbindungen der Formel VI sind an sich bekannt, oder können auf an sich bekannte Weise aus bekannten Verbindungen hergestellt werden. So kann beispielsweise eine Verbindung der allgemeinen Formel VIa, 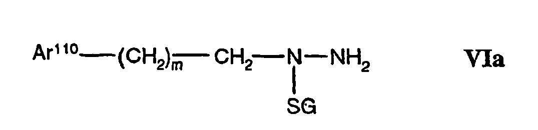 worin Ar¹¹⁰, m und SG obige Bedeutungen besitzen, auf an sich bekannte Weise durch reduktive Aminierung aus einem entsprechenden Aldehyd der allgemeinen Formel VIII und einem entsprechenden Amin der allgemeinen Formel XVII, 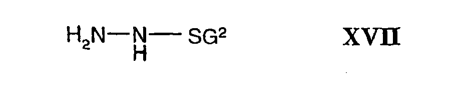 worin SG² eine in der Peptidchemie bekannte Schutzgruppe, vorzugsweise die Boc-Schutzgruppe, darstellt, nachfolgende Einführung einer Schutzgruppe SG und schließlich Abspaltung der Schutzgruppe SG² hergestellt werden. Die Umsetzung kann in einem dipolar-aprotischen Lösungsmittel wie Tetrahydrofuran (= THF) und vorzugsweise bei Raumtemperatur durchgeführt werden. Die Reduktion einer als Zwischenprodukt erhaltenen entsprechenden Iminverbindung kann in einem dipolar-aprotischen Lösungsmittel wie THF und bei Temperaturen zwischen -20 °C und Raumtemperatur, vorzugsweise bei 0 °C, durchgeführt werden. Als Reduktionsmittel eignen sich komplex Borhydride wie NaCNBH₃. Die Verbindungen der Formeln VIII und XVII sind an sich bekannt oder können auf an sich bekannte Weise aus bekannten Verbindungen hergestellt werden.

Gemäß Verfahrensvariante d) kann eine Verbindung der Formel Ij hergestellt werden, indem man eine Aminoverbindung der Formel III mit einem Aldehyd der Formel VIII umsetzt und gegebenenfalls vorhandene Schutzgruppen nachfolgend wieder abspaltet. Die Umsetzung kann in der oben für die Umsetzung von Verbindungen der Formel XVI mit Verbindungen der Formel XVII angegebenen Weise durchgeführt werden, wobei das entstandene Imin in diesem Fäll jedoch nicht reduziert wird.

Die erhaltenen Verbindungen der Formel I können jeweils auf an sich bekannte Weise aus dem Reaktionsgemisch isoliert und gereinigt werden. Säureadditionssalze können in üblicher Weise in die freien Basen überführt werden und diese können gewünschtenfalls in bekannter Weise in physiologisch verträgliche Säureadditionssalze überführt werden.

Als physiologisch verträgliche Salze von Verbindungen der Formel I kommen deren Salze mit anorganischen Säuren, beispielsweise Schwefelsäure, Phosphorsäuren oder Halogenwasserstoffsäuren, vorzugsweise Chlorwasserstoffsäure, oder mit organischen Säuren, beispielsweise niederen aliphatischen Mono-, Di- oder Tricarbonsäuren wie Maleinsäure, Fumarsäure, Milchsäure, Weinsäure, Zitronensäure, oder mit Sulfonsäuren, beispielsweise Niederalkansulfonsäuren wie Methansulfonsäure oder gegebenenfalls im Benzolring durch Halogen oder niederes Alkyl substituierte Benzolsulfonsäuren wie p-Toluolsulfonsäure, in Frage.

Die Verbindungen der Formel I können neben dem den -CH₂-Ar²-Rest tragenden Kohlenstoffatom noch weitere Chiralitätszentren enthalten, nämlich das den Substituenten R³ tragende Kohlenstoffatom, das durch C₁₋₄-Alkyl oder durch C₁₋₄-Alkyl-carbonylamid substituierte Kohlenstoffatom der Methylengruppe A³ und/oder das Kohlenstoffatom der CH-Gruppe A¹, sofern R¹ Amino bedeutet. Die Verbindungen der Formel I können somit in mehreren stereoisomeren Formen vorliegen. Die vorliegende Erfindung umfaßt sowohl die Gemische optischer Isomere als auch die isomerenreinen Verbindungen der Formel I. Bevorzugt sind isomerenreine Verbindungen der Formel I, insbesondere die Verbindungen der Formel I, worin das durch C₁₋₄-Alkyl oder durch C₁₋₄-Alkylcarbonylamid substituierte Kohlenstoffatom der Methylengruppe A³ die S-Konfiguration aufweist. Falls bei der Synthese der Verbindungen der Formel I Gemische optischer Isomere der Ausgangsverbindung eingesetzt werden, werden auch die Verbindungen der Formel I in Form von Gemischen optischer Isomere erhalten. Ausgehend von stereochemisch einheitlichen Formen der Ausgangsverbindung können auch stereochemisch einheitliche Verbindungen der Formel I erhalten werden. Die stereochemisch einheitlichen Verbindungen der Formel I können auch aus den Gemischen optischer Isomere in an sich bekannter Weise erhalten werden, z. B. durch chromatographische Trennung an chiralen Trennmaterialien oder durch Umsetzung mit geeigneten optisch aktiven Säuren, beispielsweise Weinsäure oder Campher-10-sulfonsäure, und anschließender Auftrennung in die optisch aktiven Antipoden durch fraktionierte Kristallisation der gewonnenen diastereomeren Salze.

Die neuen Verbindungen der Formel I und ihre physiologisch verträglichen Säureadditionssalze zeichnen sich durch eine hohe und im Vergleich zu den weiteren bekannten Bombesin-Rezeptorsubtypen, NMB-R und GRP-R, selektive Affinität zum Bombesin-Rezeptor des Subtyps 3 aus, an welchem sie als Agonisten wirken. Von den Verbindungen der Formel I ist daher zu erwarten, daß sie zur Prophylaxe und/oder Behandlung von Krankheitsbildern geeignet sind, welche durch eine Stimulierung des BRS-3 günstig beeinflußt werden können. Insbesondere erscheinen die erfindungsgemäßen Verbindungen zur Prophylaxe und/oder Behandlung von Fettleibigkeit (= Obesitas, Adipositas), Diabetes, Hyperinsulinämie, kardiovaskulären Erkrankungen, Essstörungen (Hyperphagie, Anorexie, Bulimie) und/oder Syndrom X geeignet.

### Beschreibung der pharmakologischen Testmethode:

Die BRS-3-agonistischen Wirkungen der Testsubstanzen können beispielsweise in einem nach der FLIPR-Methode ("Fluorometric Imaging Plate Reader") arbeitenden pharmakologischen Standardtest in vitro nachgewiesen werden.

Hierfür werden zunächst CHO Zellen (= Ovarzellen des chinesischen Hamsters, "chinese hamster ovary cells") auf an sich bekannte Weise mit einem Expressionsvektor für den Subtyp 3 des humanen Bombesin Rezeptors, d.i. BRS-3, transfiziert.

Die cDNA des humanen BRS-3 (Nukleotidsequenz unter GenBank Accession Nr. L08893) wurde mit Hilfe der Restriktionsendonuklease EcoRI aus dem Plasmidvektor pGEM4 (Fa. Promega, USA) ausgeschnitten und in den Expressionsvektor pcDNA3.1(-) (Fa. Invitrogen, USA) subkloniert. CHO-K1 Zellen, die bereits stabil mit dem Expressionsvektor RD-HGA16, der die cDNA Sequenz des humanen Gα16-Proteins (Nukleotidsequenz unter GenBank Accession Nr. M63904) trägt, transfiziert waren, wurden in Probenplatten mit 24 Probenplätzen ("24-well plate") gegeben und über Nacht unter sterilen Bedingungen im Luftbefeuchteten Inkubator bei 37 °C und 5 % CO₂ in F-12 Medium plus Glutamax-I (Fa. GibcoBRL, Kat.- Nr. 31765), welches mit 10%igem fötalem Kälberserum (inaktiviert bei 56 °C für 1 h, Fa. GibcoBRL), 25 µg/ml Gentamicin (Fa. GibcoBRL) und 0,2 mg/mL Hygromycin B (Fa. GibcoBRL) versetzt worden war, inkubiert. Am nächsten Tag wurden die Zellen mit dem Expressionsvektor für BRS-3 transfiziert, indem unter Verwendung des "Effectene Transfection Reagent" (Fa. Qiagen) je Probenplatz 12 µl einer Lösung, die 0,3 µg/µl DNA des Expressionsvektors enthielt, zugegeben wurde. Einen Tag nach der Transfektion wurde das Kulturmedium gegen Selektionsmedium ausgetauscht. Hierzu wurden die transfizierten Zellen, die jeweils gleichzeitig BRS-3 und das humane Gα16-Protein exprimieren, unter sterilen Bedingungen bei 37 °C und 5% CO₂ in F-12 Medium plus Glutamax-1 (Fa. GibcoBRL, Kat.- Nr. 31765), welches mit 10%igem fötalem Kälberserum (inaktiviert bei 56 °C für 1 h, Fa. GibcoBRL), 25 µg/ml Gentamicin (Fa. GibcoBRL), 0,2 mg/mL Hygromycin B (Fa. GibcoBRL) und 0,5 mg/ml Geneticin (Fa. GibcoBRL) versetzt worden war, kultiviert. Zur Optimierung des Zelltests wurden die Zellen mit der höchsten Rezeptorexpressionsrate ausgewählt. Dazu wurden die transfizierten Zellen mit dem oben beschriebenen Selektionsmedium 1:30000 verdünnt und in Probenplatten mit 96 Probenplätzen ("96-well plate") gegeben. Die Zellen wurden über Nacht bei 37 °C und 5% CO₂ inkubiert, anschließend wurden die Probenplätze ausgewählt, welche nur eine einzelne Zelle enthielten. Diese Zellen wurden zunächst in Probenplatten mit 24 Probenplätzen ("24-well plate") angezüchtet und dann in Costar Plastikflaschen (zuerst 25 ml und dann 225 ml) kultiviert. Die Abschätzung der BRS-3 Rezeptorexpression der jeweiligen Einzelzellklone erfolgte durch eine Bestimmung des EC₅₀-Wertes des synthetischen Nonapeptids [D-Phe⁶, β-Ala¹¹, Phe¹³ , Nle¹⁴] Bn (6-14) als Ligand (Versuchsdurchführung s.u.). Gelagert wurden die transfizierten Zellen bei -80°C in Aliquots von je 1,8 ml Medium mit 10% Dimethylsulfoxid (= DMSO) (Zell-Konzentration 1 x 10⁶ Zellen/ml). Für die Kultivierung wurde ein eingefrorenes Aliquot auf 37°C erwärmt, in eine Costar Plastikflasche (225 ml) überführt und mit 50 ml des vorstehend beschriebenen Selektionsmediums verdünnt. Das Medium wurde zunächst nach 30-minütiger Inkubation einmalig gewechselt. An jedem folgenden ersten bis dritten Tag wurde das Medium entfernt, die adhärenten Zellen (40 - 95 % Konfluenz) wurden mit PBS Dulbecco's (Fa. GibcoBRL) gewaschen und vom Flaschenboden durch 2-minütige Behandlung mit Trypsin-EDTA-Lösung (Fa. GibcoBRL) bei 37 °C gelöst. Falls die Zellen weiter kultiviert werden sollten, wurden sie in eine neue Plastikflasche mit frischem Medium überführt. Sollten mit den Zellen Experimente durchgeführt werden, wurden die Zellen in Costar Probenplatten mit 96 Probenplätzen, klarer Bodenplatte und Deckel ("Costar 96-well assay plates", Fa. Corning) überführt, nachdem die Zellkonzentration auf 1,2 x 10⁴ Zellen/ml eingestellt worden war.

BRS-3 ist über G-Proteine an den Ca²⁺-Signaltransduktionsweg der CHO-Zelle gekoppelt. Sofern ein Agonist an den Rezeptor bindet, wird über das G-Protein die Phospholipase C aktiviert, welche dann ihrerseits die Synthese wasserlöslicher Inositolphosphate katalysiert. Diese wasserlöslichen Inositolphosphate verursachen die Freisetzung von Ca²⁺, welches im endoplasmatischen Retikulum gespeichert ist. Die transiente Erhöhung der cytosolischen Ca²⁺ Konzentration wurde im sog. FLIPR-Experiment gemessen. Hierzu wurden die Zellen mit einem Ca²⁺-bindenden, fluoreszierenden Farbstoff, Fluo4 (Fa. Molecular Probes) beladen. Dieser intrazelluläre Farbstoff bindet die nach Aktivierung freigesetzten cytosolischen Ca²⁺-Ionen und verstärkt dabei seine Fluoreszenzintensität. Die Änderung der Fluoreszenzintensität ist proportional zur Änderung der intrazellulären Ca²⁺ Konzentration und ist ein Maß für die Aktivierung der Zelle durch den entsprechenden Agonisten. Unterhalb der maximalen Fluoreszenzantwort ist der Grad der Aktivierung abhängig von der Konzentration der eingesetzten Verbindungen. Die Fluoreszenzänderung durch Aktivierung von BRS-3 wurde für jede zu testende Substanz bei verschiedenen Substanz-Konzentration bestimmt. Als Referenzwert für eine 100%ige Aktivierung diente die maximale Fluoreszenz-Antwort bei Aktivierung des BRS-3 mit dem synthetischen Nonapeptid [D-Phe',β-Ala",Phe",Nle"1]Bn(6-14) [vgl. Mantey et al. (1997) J. Biol. Chem. 272: 26062-26071]. Die Konzentration der Verbindung, bei der eine 50%ige Aktivierung eintrat, wurde als EC₅₀ Wert bestimmt und diente als Maß für die Wirksamkeit der jeweiligen Testverbindung als BRS-3-Agonist.

Die transfizierten CHO-Zellen wurden 18 bis 24 Stunden (= h) in den "Costar 96-well assay plates" (Fa. Corning) kultiviert, bis sie konfluent waren. Eine 250 mM Stamm-Lösung von Probenecid wurde jeden Tag frisch hergestellt. Dazu wurden 710 mg Probenecid (Fa. Sigma # P8761), in 5 ml 1 N NaOH gelöst und anschließend mit HBSS Medium ohne Phenolrot (GibcoBRL), welches 20 mM HEPES (Fa. PAA Laboratories) enthielt, auf 10 ml verdünnt. Eine 2 mM Stammlösung des fluoreszierenden Calciumionen-Indikatorfarbstoffes Fluo4 wurde durch Lösen von 1 mg Fluo4 in 440 µl DMSO hergestellt und bei -20 °C aufbewahrt. Außerdem wurde eine 20%ige (w/v) Lösung von Pluronic F-127 (Fa. Sigma) in DMSO verwendet. Unmittelbar vor Verwendung wurde ein 22 µl Aliquot der Fluo4 Stammlösung aufgetaut.

Das Beladungsmedium wurde stets frisch hergestellt, indem 42 ml HBSS Medium ohne Phenolrot (GibcoBRL), das 60 mM HEPES (Fa. PAA Laboratories) enthielt, mit 420 µl der Probenecid Stammlösung und je 22 µl Fluo4 Stammlösung und Pluronic F-127-Lösung gemischt wurden. Die Zellen wurden je Probenplatz mit 100 µl frischem Beladungsmedium für 45 - 60 min bei 37 °C und 5 % CO₂ inkubiert. Danach wurden die Zellen dreimal mit je 100 µl HBSS Medium mit 20 mM HEPES und 2.5 mM Probenecid gewaschen. Im Anschluss an den letzten Waschschritt verblieben in jedem der 96 Probenplätze 100 µl Volumen auf den Zellen.

Von den Verbindungen der Formel I wurden jeweils 10 mM Stammlösungen in DMSO hergestellt, von denen in Mikrotiterplatten mit 96 Probenplätzen ("96-well plates", Fa. Greiner) Verdünnungsreihen mit HBSS Medium mit 20 mM HEPES angelegt wurden. Die höchste in den Messungen verwendete Konzentration war üblicherweise 33 µM, in einigen Fällen aber auch nur 1 µM. Die Lösungen wurden in Abhängigkeit von der jeweiligen Verbindung 1:2, 1:3, 1:4 oder 1:10 auf 8 oder 16 verschiedene Probenplätze verdünnt. Als Referenz enthielt jede Mikrot.iterplatte eine Verdünnungsreihe des Nonapeptids [D-Phe⁶, β-Ala¹¹, Phe¹³, Nle¹⁴] Bn (6-14).

Das FLIPR Gerät (Fa. Molecular Devices) wurde so programmiert, dass es die Hintergrund-Fluoreszenz über einen Zeitraum von 30 Sekunden (= Sek.) in 6-Sek.-Intervallen maß. Nach dem Transfer von jeweils 50 µl aus jedem Probenplatz der Mikrotiterplatte in den entsprechenden Probenplatz der Zellplatte wurde die Änderung der Fluoreszenz über einen Zeitraum von 100 Sekunden (= Sek.) in 1-Sek.-Intervallen aufgezeichnet, und in 6-Sek.-Intervallen während der letzten 42 Sek.

Die Fluoreszenzänderungen der Referenzverbindung in Abhängigkeit von der Konzentration wurden aufgezeichnet, und die Peptid-Konzentration des Nonapeptids, bei der bereits die maximale Fluoreszenzänderung beobachtet wurde, wurde bestimmt (meist 16 µM). Der Wert der maximalen Fluoreszenzänderung je Probenplatz wurde an das Tabellenkalkulationsprogramm Excel (Fa. Microsoft) exportiert und mit Hilfe des Maximalwertes der Fluoreszenzänderung für die entsprechende Referenzverbindung, welcher als 100%-Wert angenommen wurde, normalisiert. Die Kurven für den Verlauf der relativen Fluoreszenzänderung in Abhängigkeit von der Konzentration der zu untersuchenden Verbindung und der entsprechende EC₅₀-Wert wurden mit Hilfe des Programmes Graphpad Prism (Version 3.00, Fa. Graphpad Software) berechnet.

In dem vorstehend beschriebenen pharmakologischen FLIPR-Test zeigten alle nachfolgend angegebenen Beispielsverbindungen EC_{5O}-Werte (in nM), welche kleiner oder gleich 2600 waren. Die Verbindungen der Beispiele 13 bis 34 zeigten EC₅₀-Werte, welche kleiner oder gleich 710 waren. In der nachfolgenden Tabelle 1 sind die im oben beschriebenen FLIPR-Experiment ermittelten EC₅₀-Werte für einzelne Verbindungen der Formel I aufgeführt. Die in der Tabelle 1 angegebenen Beispielsnummern beziehen sich auf die nachstehenden Herstellungsbeispiele.

**Tabelle 1: Agonistische Aktivität der Testsubstanzen an BRS-3**

| **Beispiel-Nr.** | **EC₅₀ [nM]** |
|---|---|
| 2 | 2,1 |
| 3 | 4,0 |
| 4 | 1,4 |
| 5 | 6,0 |
| 6 | 1,5 |
| 7 (Vergleichsbeispiel) | 30 |
| 15 | 57 |
| 19 | 32 |
| 21 | 21 |
| 22 | 2,9 |
| 23 | 21 |
| 24 | 17 |
| 26 | 25 |
| 27 | 3,1 |
| 28 | 0,19 |
| 30 | 2,2 |

Die Verbindungen der Formel I können in üblichen pharmazeutischen Zubereitungen verabreicht werden. Die zu verwendenden Dosen können individuell verschieden sein und variieren naturgemäß je nach Art des zu behandelnden Zustandes und der verwendeten Substanz. Im allgemeinen eignen sich zur Applikation am Menschen und an größeren Säugetieren jedoch Arzneiformen mit einem Wirkstoffgehalt von 0,1 bis 300 mg pro Einzeldosis.

Die Verbindungen der Formel I können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt oder auch Suppositorien. Diese Präparate können pharmazeutisch übliche anorganische und/oder organische Trägerstoffe, wie z. B. Talkum, Milchzucker oder Stärke, neben pharmazeutisch üblichen Hilfsmitteln, beispielsweise Gleitmitteln oder Tablettensprengmitteln, enthalten. Flüssige Präparate wie Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die nachfolgend angeführten Beispiele sollen die Erfindung näher erläutern, ohne sie in ihrem Umfang zu beschränken.

### Beispiel 1:

### N1-[(1R)-2-(1H-3-indolyl)-1-(phenethylcarbamoyl)-ethyl]-(2S)-2-{[(1R)-1-amino-2-phenethyl]-carboxamido}-pentandiamid; (H-D-Phe-Gln-D-Trp-Phenylethylamid)

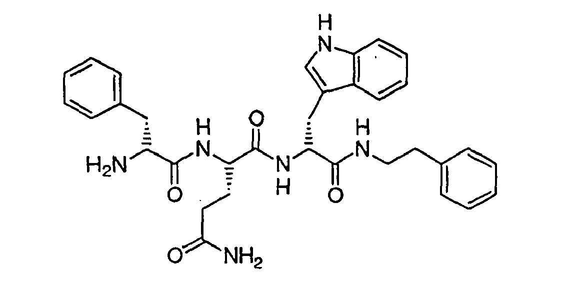

A) 100 mg FMPE-Harz (maximale Kapazität 0,54 mmol/g) ließ man in 1 ml Dichlorethan 10 Min. lang quellen. Zu dieser Vorlage gab man 0,5 ml Trimethylorthoformat (= TMOF), 68 µl 2-Phenylethylamin und 114 mg NaBH(OAc)₃ zu, behandelte die entstandene Mischung 10 Min. lang mit Ultraschall und schüttelte diese anschließend noch über Nacht bei RT. Dann wusch man das Harz der Reihe nach dreimal drei Min. lang mit je 3 ml Dichlormethan und dreimal drei Min. lang mit je 3 ml NMP. Nun gab man zu dem gewaschenen Harz eine Lösung von 56 mg Fmoc-D-Trp(Boc)-OH, 41 mg HATU, 14,6 mg HOAt und 143 µl sym.-Collidin in 1 ml NMP zu. Man schüttelte das Harz in dieser Lösung 5 h lang bei RT, wusch dreimal drei Min. lang mit je 3 ml NMP und behandelte das Harz über Nacht erneut mit einer Lösung von 56 mg Fmoc-D-Trp(Boc)-OH, 41 mg HATU, 14,6 mg HOAt und 143 µl sym.-Collidin in 1 ml NMP. Schließlich wusch man das Harz dreimal drei Min. lang mit je 1 ml Dichlormethan und trocknete im Ölpumpenvakuum. Man erhielt 129 mg eines mit Fmoc-D-Trp(Boc)-Phenylethylamid beladenen FMPE-Harzes [Beladung 0,366 mmol/g; entsprechend 30 mg (0,047 mmol) freien Fmoc-D-Trp(Boc)-Phenylethylamids], welches ohne Abspaltung des Zwischenproduktes direkt für die nachfolgend angegebene Umsetzung eingesetzt wurde.
B) Die gesamte Menge des vorstehend erhaltenen beladenen FMPE-Harzes [beladen mit 0,366 mmol/g Fmoc-D-Trp(Boc)-Phenylethylamid, entsprechend 30 mg (0,047 mmol) der freien Verbindung] ließ man 10 Min. lang in 5 ml NMP quellen. Dann behandelte man das FMPE-Harz 15 Min. lang mit 5 ml einer frisch bereiteten 20%-igen (v/v) Lösung von Piperidin in NMP, wusch fünfmal drei Min. lang mit je 5 ml NMP und behandelte das FMPE-Harz schließlich erneut 15 Min. lang mit 5 ml einer frisch bereiteten 20%igen (v/v) Lösung von Piperidin in NMP. Zuletzt wurde das Harz fünfmal drei Min. lang mit je 5 ml NMP gewaschen. Das erhaltene, mit D-Trp(Boc)-Phenylethylamid beladene FMPE-Harz wurde ohne Isolierung des Zwischenproduktes direkt für die nachfolgend angegebene Umsetzung eingesetzt.
C) Die gesamte Menge des vorstehend erhaltenen beladenen FMPE-Harzes [bei angenommener 100%iger Umsetzung beladen mit 0,366 mmol/g D-Trp(Boc)-Phenylethylamid, entsprechend 19,2 mg (0,047 mmol) der freien Verbindung] wusch man fünfmal drei Min. lang mit je 5 ml NMP. Anschließend gab man zu dem beladenen FMPE-Harz eine Lösung von 57,4 mg Fmoc-Gln(Trt)-OH, 12,7 mg HOBT×H₂O und 30 mg TBTU in 2 ml NMP hinzu. Schließlich gab man zu der entstandenen Vorlage noch 46 µl DIPEA zu und schüttelte 45 Min. lang. Nachdem das FMPE-Harz fünfmal drei Min. lang mit je 5 ml NMP gewaschen worden war, wiederholte man den vorstehend beschriebenen Kopplungsschritt. Abschließend wusch man nochmals fünfmal drei Min. lang mit je 5 ml NMP. Man erhielt ein mit Fmoc-Gln(Trt)-D-Trp(Boc)-Phenylethylamid beladenes FMPE-Harz, welches ohne Isolierung des Zwischenproduktes direkt für die nachfolgend angegebene Umsetzung eingesetzt wurde.
D) Die gesamte Menge des vorstehend erhaltenen beladenen FMPE-Harzes [bei angenommener 100%iger Umsetzung beladen mit 0,366 mmol/g Fznoc-Gln(Trt)-D-Trp(Boc)-Phenylethylamid, entsprechend 47 mg (0,047 mmol) der freien Verbindung] wurden zur Abspaltung der Fmoc-Schutzgruppe wie vorstehend unter B) beschrieben behandelt. Man erhielt ein mit Gln(Trt)-D-Trp(Boc)-Phenylethylamid beladenes FMPE-Harz, welches ohne Isolierung des Zwischenproduktes direkt für die nachfolgend angegebene Umsetzung eingesetzt wurde.
E) Die gesamte Menge des vorstehend erhaltenen beladenen FMPE-Harzes [bei angenommener 100%iger Umsetzung beladen mit 0,366 mmol/g Gln(Trt)-D-Trp(Boc)-Phenylethylamid, entsprechend 36,6 mg (0,047 mmol) der freien Verbindung] wusch man fünfmal drei Min. lang mit je 5 ml NMP. Anschließend gab man zu dem beladenen FMPE-Harz eine Lösung von 36,4 mg Fmoc-Phe-OH, 12,7 mg HOBT×H₂O und 30 mg TBTU in 2 ml NMP hinzu. Schließlich gab man zu der entstandenen Vorlage 46 µl DIPEA zu und schüttelte 45 Min. lang. Nachdem das FMPE-Harz fünfmal drei Min. lang mit je 5 ml NMP gewaschen worden war, wiederholte man den vorstehend beschriebenen Kopplungsschritt. Abschließend wusch man nochmals fünfmal drei Min. lang mit je 5 ml NMP. Man erhielt ein mit Fmoc-Phe-Gln(Trt)-D-Trp(Boc)-Phenylethylamid beladenes FMPE-Harz, welches ohne Isolierung des Zwischenproduktes direkt für die nachfolgend angegebene Umsetzung eingesetzt wurde.
F) Die gesamte Menge des vorstehend erhaltenen beladenen FMPE-Harzes [bei angenommener 100%-iger Umsetzung beladen mit 0,366 mmol/g Fmoc-Phe-Gln(Trt)-D-Trp(Boc)-Phenylethylamid, entsprechend 54 mg (0,047 mmol) der freien Verbindung] wurden zur Abspaltung der Fmoc-Schutzgruppe wie vorstehend unter B) beschrieben behandelt. Man erhielt ein mit Phe-Gln(Trt)-D-Trp(Boc)-Phenylethylamid beladenes FMPE-Harz, welches ohne Isolierung des Zwischenproduktes direkt für die nachfolgend angegebene Umsetzung eingesetzt wurde.
G) Die gesamte Menge des vorstehend erhaltenen beladenen FMPE-Harzes [bei angenommener 100%iger Umsetzung beladen mit 0,366 mmol/g Phe-Gln(Trt)-D-Trp(Boc)-Phenylethylamid, entsprechend 43,5 mg (0,047 mmol) der freien Verbindung] wusch man dreimal zehn Min. lang mit je 3 ml Dichlormethan. Anschließend behandelte man das beladene FMPE-Harz dreimal 30 Min. lang mit je 2 ml einer Mischung aus Trifluoressigsäure (= TFA)/Triisopropylsilan (= TIPS)/H₂0 (18:1:1 v/v/v) und filtrierte jeweils vom FMPE-Harz ab. Anschließend wurde erneut dreimal drei Min. lang mit je 3 ml Dichlormethan gewaschen und vom FMPE-Harz abfiltriert. Die vereinigten Filtrate wurden im Wasserstrahlpumpenvakuum mit stickstoffgekühlter Vorlage eingedampft. Der verbleibende Rückstand wurde in DMSO aufgenommen und durch Reversed-Phase-HPLC [HPLC-System von Amersham *Pharmacia Biotech* Äkta Basic 100F; Pumpensystem P-900 und Detektor UV-900; Säule ODS-A C₁₈ von *Omnicrom YMC* (250 mm X 20 mm, 10 µm. Flußrate: 8 ml/min); Elution mit linearem Gradienten (30 Min.) von Wasser (Solvens A) in Acetonitril (Solvens B) und 0.1 % (v/v) TFA] gereinigt. Lyophilisierung der gereinigten Fraktionen lieferte 19,2 mg der Titelverbindung als farbloses Pulver.
   HPLC-MS (ESI) m/z 276.1 (32), 308.1 (90), 583.3 (72) [m+H]⁺, 605.4 (100) [m+Na]⁺, 893.6 (13), 1165.2 (10) [2m+H]⁺, 1187.2 (40) [2m+Na]⁺.

### Beispiel 2:

### N1-Phenethyl-(2R)-2-[(1S)-1-(benzylcarboxamido)-ethyl]-carboxamido-3-(1H-3-indolyl)-propanamid

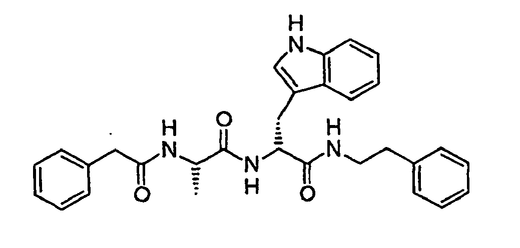

A) 100 mg eines mit Fmoc-D-Trp(Boc)-Phenylethylamid beladenen FMPE-Harzes [Herstellung siehe Beispiel 1A); Beladung 0,354 mmol/g; entsprechend 22,3 mg (0,035 mmol) freien Fmoc-D-Trp(Boc)-Phenylethylamids] wurden in der in Beispiel 1B entsprechenden Weise umgesetzt. Das entstandene, harzgebundene Trp(Boc)-Phenylethylamid wurde ohne Isolierung oder Reinigung direkt für die nachfolgend angegebene Umsetzung eingesetzt.
B) Die gesamte Menge des vorstehend erhaltenen, mit D-Trp(Boc)-Phenylethylamid beladenen FMPE-Harzes [bei angenommener 100%iger Umsetzung beladen mit 0,354 mmol/g D-Trp(Boc)-Phenylethylamid, entsprechend 14,4 mg (0,035 mmol) der freien Verbindung] wusch man fünfmal drei Min. lang mit je 5 ml NMP. Anschließend gab man zu dem beladenen FMPE-Harz eine Lösung von 22 mg Fmoc-Ala-OH, 9,5 mg HOBT×H₂O und 22,5 mg TBTU in 2 ml NMP hinzu. Schließlich gab man noch 34 µl DIPEA hinzu und schüttelte das entstandene Gemisch 45 Min. lang. Nachdem das FMPE-Harz fünfmal drei Min. lang mit je 5 ml NMP gewaschen worden war, wiederholte man den vorstehend beschriebenen Kopplungsschritt. Abschließend wusch man nochmals fünfmal drei Min. lang mit je 5 ml NMP. Man erhielt ein mit Fmoc-Ala-D-Trp(Boc)-Phenylethylamid beladenes FMPE-Harz, welches ohne Isolierung direkt für die nachfolgend angegebene Umsetzung eingesetzt wurde.
C) Die gesamte Menge des vorstehend erhaltenen beladenen FMPE-Harzes [bei angenommener 100%iger Umsetzung beladen mit 0,354 mmol/g Fmoc-Ala-D-Trp(Boc)-N-Phenylethylamid, entsprechend 24,5 mg (0,035 mmol) der freien Verbindung] wurden zur Abspaltung der Fmoc-Schutzgruppe wie vorstehend unter Beispiel 1B) beschrieben behandelt. Man erhielt ein mit Ala-D-Trp(Boc)-Phenylethylamid beladenes FMPE-Harz, welches ohne Isolierung direkt für die nachfolgende Umsetzung eingesetzt wurde.
D) Die gesamte Menge des vorstehend erhaltenen beladenen FMPE-Harzes [bei angenommener 100%-iger Umsetzung beladen mit 0,354 mmol/g Ala-D-Trp(Boc)-Phenylethylamid, entsprechend 16,7 mg (0,035 mmol) der freien Verbindung] wusch man fünfmal drei Min. lang mit je 5 ml NMP. Anschließend gab man zu dem beladenen FMPE-Harz eine Lösung von 9,6 mg Phenylessigsäure, 9,5 mg HOBT×H₂O und 22,5 mg TBTU in 2 ml NMP hinzu. Schließlich gab man noch 34 µl DIPEA zu und schüttelte das entstandene Gemisch 45 Min. lang. Nachdem das FMPE-Harz fünfmal drei Min. lang mit je 5 ml NMP gewaschen worden war, wiederholte man den vorstehend beschriebenen Kopplungsschritt. Abschließend wusch man nochmals fünfmal drei Min. lang mit je 5 ml NMP. Man erhielt ein mit Phenylacetat-Ala-D-Trp(Boc)-Phenylethylamid beladenes FMPE-Harz, welches ohne Isolierung direkt für die nachfolgende Umsetzung eingesetzt wurde.
E) Die gesamte Menge des vorstehend erhaltenen beladenen FMPE-Harzes [bei angenommener 100%iger Umsetzung beladen mit 0,354 mmol/g Phenylacetat-Ala-D-Trp(Boc)-N-Phenylethylamid, entsprechend 20,9 mg (0,035 mmol) der freien Verbindung] wurden zur Abspaltung vom FMPE-Harz und Entfernung der Boc-Schutzgruppe wie vorstehend in Beispiel 1G) beschrieben behandelt. Reinigung des erhaltenen Rohproduktes mittels HPLC und anschließende Lyophilisierung lieferten 12,6 mg (0,025 mmol) der Titelverbindung als farbloses Pulver mit einem Schmelzpunkt von 205-207 °C. ¹H-NMR (500 MHz, DMSO-d₆, 300 K) δ = 10.78 (s, 1*H*, N*H*-CH-C), 8.24 (d, *J*_{HH} = 6.8 Hz, 1H, NH-CH-CH₃), 8.18 (d, *J* = 8.4 Hz, 1H, N*H*-CH-CH₂), 8.00 (t, *J* = 5.5 Hz, 1H, N*H*-CH₂-CH₂), 7.57 (d, *J* = 7.9 Hz, 1H, arom), 6.95-7.32 (m, 14H, arom), 4.38-4.43 (m, 1H, NH-CH-CH₂), 4.21-4.25 (m, 1H, NH-C*H*-CH₃), 3.45 (s, 2H, CO-C*H*2), 3.19-3.24 (m, 2H, NH-C*H*₂-CH₂), 3.11 (dd, *J*=14.7Hz, *J*=4.6Hz, 1H, NH-CH-C*H*₂), 2.84 (dd, *J* = 14.6 Hz, *J* = 9.6 Hz, 1H, NH-CH-C*H*₂), 2.61 (t, *J* = 7.6 Hz, 2H, NH-CH₂-C*H*₂), 1.01 (d, *J* = 7.0 Hz, 3H, C*H*₃).
   **HPLC-MS** (ESI) m/z 159.1 (40), 291.2 (35), 308.1 (100), 497.2 (80) [M+H]⁺, 519.4 (55) [M+Na]⁺, 764.5 (20), 993.1 (10) [2M+H]⁺, 1015.2 (100) [2M+Na]⁺.

### Beispiel 3:

### N1-Phenethyl-(2R)-2-{1-[(4-chlorbenzyl)-amino]-ethylcarbox-amido}-3-(1H-3-indolyl)-propanamid

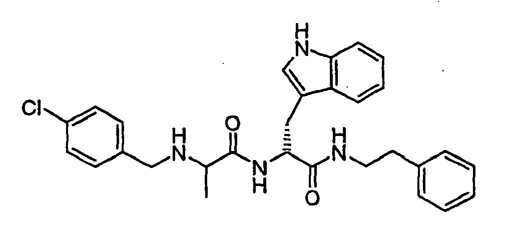

A) Zu einer Lösung von 7,08 g 4-Chlorbenzylamin und 5,06 g Triethylamin in 38 ml Toluol wurde unter Rühren und Eiskühlung-über einen Zeitraum von 4 h tropfenweise eine Lösung von 8,35 g 2-Brompropionsäureethylester in 18 ml Toluol zugegeben und man ließ das Reaktionsgemisch anschließend 4 Tage lang rühren. Dann extrahierte man die organische Phase mit 300 ml Wasser und trocknete sie anschließend über Na₂SO₄. Das Lösungsmittel wurde im Wasserstrahlpumpenvakuum eingedampft und der erhaltene Rückstand wurde mittels Flash-Chrömatographie unter einem Druck von 1-1,2 bar (stationäre Phase: Silicagel 60, Korngröße 0,040-0,063 mm, mobile Phase: Essigsäureethylester/Hexan 1:1) gereinigt. Nach Einengen des Lösungsmittels im Wasserstrahlpumpenvakuum und Trocknung des Rückstandes im Ölpumpenvakuum erhielt man 4,25 g N-(4-Chlorbenzyl)-alaninethylester als gelbes Ö1. **¹H-NMR** (250 MHz, DMSO-d₆, 300 K) δ = 7.31-7.38. (m, 4H, arom), 4.09 (q, *J* = 7.1 Hz, 2H, C*H*₂-CH₃), 3.65 (q, *J* = 13.9 Hz, NH-C*H*₂-C₆H₄Cl), 3.20-3.24 (m, 1H, NH-CH), 2.51 (bs, 1H, N*H*), 1.17-1.22 (m, 6H, CH₂-CH₃ and CH-C*H*₃).
B) 1,0 g des vorstehend erhaltenen N-(4-Chlorbenzyl)-alaninethylesters wurden mit 6,2 ml 1 N NaOH und 12 ml Methanol versetzt und 30 Min. lang gerührt. Man neutralisierte mit 1 N HCl und dampfte das Lösungsmittel im Wasserstrahlpumpenvakuum ein. Der erhaltene Rückstand wurde in einer Mischung aus 15 ml gesättigter wässriger NaHCO₃-Lösung und 4 ml Dioxan aufgenommen. Zu dieser Vorlage gab man über einen Zeitraum von 15 Min. und unter Eiskühlung tropfenweise eine Lösung von 1,1 g FmocCl in 8 ml Dioxan. Das Reaktionsgemisch wurde 30 Min. lang unter Eiskühlung und anschließend über Nacht bei RT gerührt. Dann versetzte man das Reaktionsgemisch mit 20 ml Wasser, trennte die wässrige Phase ab und extrahierte sie mit 100 ml Diethylether. Die wässrige Phase wurde durch Zugabe von konzentrierter Salzsäure auf pH 1 eingestellt und anschließend erneut dreimal mit je 100 ml Essigsäureethylester extrahiert. Die vereinigten organischen Phasen wurden über Na₂SO₄ getrocknet und das Lösungsmittel wurde im Wasserstrahlpumpenvakuum eingedampft. Der erhaltene Rückstand wurde säulenchromatographisch gereinigt (stationäre Phase: Silicagel 60, Korngröße 0,040-0,063 mm, mobile Phase: Essigsäureethylester/Hexan/Essigsäure 1:1:1). Nach Eindampfen des Lösungsmittels im Wasserstrahlpumpenvakuum und Trocknen des Rückstandes im Ölpumpenvakuum erhielt man 1,3 g 2-{4-Chlorbenzyl-[(9*H*-fluoren-9-ylmethoxy)-carbonyl]-amino}-propansäure als farbloses Öl (2,98 mmol). Das Verhältnis der cis/trans-Isomeren wurde nicht bestimmt.
   HPLC-MS (ESI) m/z 179.1 (95), 436.0 (75) [M+H]⁺, 458.2 (40) [M+Na]⁺, 893.0 (50) [2M+Na]⁺, 909.2 (100) [2M+K]⁺.
C) 0,26 g Phenylethylamin, 1,13 g Fmoc-D-Trp(Boc)-OH, 0,43 g HOBT und 1,03 g TBTU wurden in 15 ml DMF gelöst. Zu dieser Vorlage wurden über einen Zeitraum von 5 Min. 1,19 g DIPEA hinzugetropft. Dann ließ man das Reaktionsgemisch 1 h lang rühren, dampfte das Lösungsmittel im Wasserstrahlpumpenvakuum ein und reinigte den verbliebenen Rückstand mittels Flash-Chromatographie unter einem Druck von 1-1,2 bar (stationäre Phase: Silicagel 60, Korngröße 0,040-0,063 mm, mobile Phase: Essigsäureethylester/Hexan 1:1). Nach Eindampfen des Lösungsmittels im Wasserstrahlpumpenvakuum und Trocknen des Rückstandes im Ölpumpenvakuum erhielt man 1,33 g *N*1-Phenethyl-(2*R*)-2-(9*H*-fluoren-9-ylmethoxy)-carboxamido-3-[1-(*tert*-butoxycarbonyl)-3-indolyl]-propanamid (= Fmoc-D-Trp(Boc)-Phenylethylamid) als farblosen, wachsartigen Feststoff mit einem Schmelzpunkt von 140°C, HPLC-MS (ESI) m/z 308.2 (20), 530.3 (40), 630.3 (40) [M+H]⁺, 652.4 (10) [M+Na]⁺, 1259.5 (100) [2M+H]⁺, 1281.5 (30) [2M+Na]⁺.
D) 1,0 g des vorstehend erhaltenen Fmoc-D-Trp(Boc)-Phenyl-ethylamids wurden in 6 ml einer 20%-igen (v/v) Lösung von Piperidin in DMF gelöst. Das Reaktionsgemisch wurde 30 Min. lang gerührt und das Lösungsmittel wurde anschließend im Wasserstrahlpumpenvakuum mit stickstoffgekühlter Vorlage abgedampft. Der verbliebene Rückstand wurde mittels Flash-Chromatographie unter einem Druck von 1-1,2 bar (stationäre Phase: Silicagel 60, Korngröße 0,040-0,063 mm, mobile Phase:Essigsäureethylester/Hexan 2:1) von dem entstandenen Fmoc-Piperidin-Komplex abgetrennt und anschließend eluiert (mobile Phase: Chloroform/ Methanol 15:1). Einengen des Lösungsmittels im Wasserstrahlpumpenvakuum und Trocknen des Rückstandes im Ölpumpenvakuum lieferte 0,63 g *tert*-Butyl-3-[(2*R*)-2-amino-2-(phenethylcarbamoyl)-ethyl]-1*H*-1-indolcarboxylat (=D-Trp(Boc)-Phenylethylamid) als gelbes Ö1.
   MS (ESI) m/z 159.1 (20), 291.2 (75), 308.1 (95), 352.1 (70), 408.1 (100) [M+H]⁺, 430.1 (35) [M+Na]⁺, 815.2 (15) [2M+H]⁺, 837.1 (20) [2M+Na]⁺.
E) 0,32 g 2-{4-Chlorbenzyl-[(9*H*-fluoren-9-ylmethoxy)-carbonyl]-amino}-propansäure (0.736 mmol, Herstellung siehe oben unter B), 0,30 g *tert*-Butyl-3-[(2*R*)-2-amino-2-(phenethylcarbamoyl)ethyl]-1*H*-2-indol-carboxylat, Herstellung siehe oben unter D), 0,42 g HATU und 0,15 g HOAt wurden in 7 ml DMF gelöst. Dann fügte man tropfenweise über einen Zeitraum von 10 Min. 1,33 g sym.-Collidin hinzu. Man ließ das Reaktionsgemisch 1 h lang rühren und dampfte dann das Lösungsmittel im Wasserstrahlpumpenvakuum unter Stickstoffkühlung ab. Der verbliebene Rückstand wurde mittels Flash-Chromatographie unter einem Druck von 1-1,2 bar (stationäre Phase: Silicagel 60, Korngröße 0,040-0,063 mm, mobile Phase: Essigsäureethylester/Hexan 1:1) gereinigt. Einengen des Lösungsmittels im Wasserstrahlpumpenvakuum und Trocknen des Rückstandes im Ölpumpenvakuum lieferte 0,54 g *N*1-Phenethyl-(2*R*)-2-{1-[*N*-(4-chlorbenzyl)-*N*-(9*H*-fluoren-9-ylmethoxycarbonyl)-amino]-ethylcarboxamido}-3-[1-(*tert*-butoxycarbonyl)-3-indolyl]-propanamid als farblosen Schaum
   MS (ESI) m/z 179.2 (10), 769.4 (10), 825.4 (100) [m+H]⁺, 1651.6 (55) [2m+H]⁺.
F) Die gesamte Menge (0,54 g) des vorstehend erhaltenen *N*1-Phenethyl-(2*R*)-2-{1-[*N*-(4-chlorbenzyl)-*N*-(9*H*-fluoren-9-ylmethoxycarbonyl)-amino]-ethylcarboxamido}-3-[1-(*tert-*butoxycarbonyl)-3-indolyl]-propanamids (0,654 mmol) wurden in 2,5 ml Dichlormethan gelöst. Zu dieser Vorlage gab man 0,25 ml Triisopropylsilan hinzu und kühlte die entstandene Mischung auf 0 °C. Dann wurde die Mischung über einen Zeitraum von 5 Min. tropfenweise mit 2,5 ml TFA versetzt und für 1 h bei 0 °C gerührt. Das Lösungsmittel wurde im Wasserstrahlpumpenvakuum mit stickstoffgekühlter Vorlage eingedampft. Der verbliebene Rückstand wurde in einer Mischung aus 20 ml DMSO, 2,5 ml Wasser und 2,5 ml Essigsäure aufgenommen und über Nacht bei RT gerührt. Dann wurde das Lösungsmittel im Wasserstrahlpumpenvakuum eingedampft und das als Rückstand verbliebene *N*1-Phenethyl-(2*R*)-2-{1-[*N*-(4-chlorbenzyl)-*N*-(9*H*-fluoren-9-ylmethoxycarbonyl)-amino]-ethylcarboxamido}-3-[1*H*-3-indolyl]-propanamid wurde ohne weitere Reinigung oder Charakterisierung direkt für die nachfolgend angegebene Umsetzung eingesetzt.
G) Die gesamte Menge des vorstehend erhaltenen Fmoc-geschützten Propanamids (0,654 mmol bei 100%iger Umsetzung) wurde in 10 ml einer 20%igen (v/v) Lösung von Piperidin in DMF gelöst und 30 Min. lang gerührt. Das Lösungsmittel wurde im Wasserstrahlpumpenvakuum mit stickstoffgekühlter Vorlage eingedampft und der erhaltene Rückstand wurde mittels Flash-Chromatographie unter einem Druck von 1-1,2 bar (stationäre Phase: Silicagel 60, Korngröße 0,040-0,063 mm, mobile Phase: Essigsäureethylester) gereinigt. Nach Lyophilisierung der gereinigten Fraktionen erhielt man 320 mg der Titelverbindung (0,637 mmol) als farbloses Pulver vom Schmelzpunkt 107-110 °C. Das Verhältnis der beiden Isomeren zueinander betrug 1:1,24. **¹H-NMR** (500 MHz, DMSO-*d*₆, 300 K) δ = 10.85 and 10.83 (s, 1H, N*H*-C*H*-C), 9.2 (m, 1H, N*H*-CH-CH₃), 8.72 and 8.77 (d, *J* = 8.5 Hz, 1H, N*H*-CH-CH₂), 8.28 and 8.34 (t, *J* = 5.5 Hz, 1H, N*H*-CH₂-CH₂), 7.68 and 7.63 (d, *J* = 7.7 Hz, 1H, arom), 6.98-7.48 (m, 13H, arom), 4.61-4.69 (m, 1H, NH-C*H*), 3.98-4.02 (m, 1H, NH-C*H*₂-C₆H₄Cl), 3.75 (m, 1H, NH-C*H-*CH₃), 3.60-3.67 and 3.39-3.43 (m, 1H, NH-C*H*₂-C₆H₄Cl), 3.34-3 .38 (m, 1H, NH-C*H*₂-CH₂), 3.24-3.29 (m, 1H, NH-C*H*₂-CH₂), 3.05-3.08 (m, 1H, NH-CH-C*H*₂), 2.85-2.92 (m, 1H, NH-CH-C*H*₂), 2.67-2.71 (m, 2H, NH-CH₂-C*H*₂), 1.33 and 1.10 (d, *J* = 6.9 Hz, 3H, C*H*₃).
   **HPLC-MS** (ESI) m/z 291.2 (30), 308.1 (100), 503.2 (35) [M+H]⁺, 525.4 (15) [M+Na]⁺, 1027.1 (20) [2M+Na]⁺.

### Beispiel 4:

### N1-Phenethyl-(2R)-2-{N'-[2-(3-pyridyl)-ethanoyl]-hydrazino}-carboxamido-3-(1H-3-indolyl)-propanamid (181)

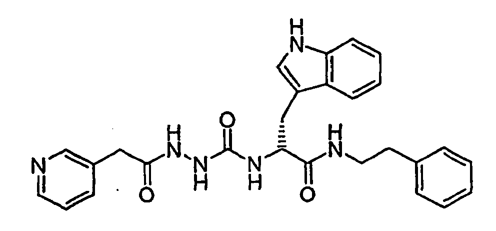

A) In 200 ml trockenem Dichlormethan und 12,95 ml DIPEA (75,6 mmol) löste man 10,0 g Boc-Hydrazin und kühlte die Lösung anschließend auf 0 °C ab. Zu dieser Vorlage wurde über einen Zeitraum von 30 Min. eine Lösung von 19,6 g FmocCl in 100 ml trockenem Dichlormethan zugetropft. Dann ließ man das Reaktionsgemisch über Nacht bei RT rühren. Im Anschluss wurde die organische Phase mit 200 ml Wasser extrahiert, über Na₂SO₄ getrocknet und im Wasserstrahlpumpenvakuum auf ein Volumen von 100 ml eingeengt. Dann gab man unter Eiskühlung 100 ml Trifluoressigsäure hinzu und ließ die Mischung für 1,5 h rühren. Man gab 300 ml gesättigter wässriger Na₂CO₃-Lösung zu der Mischung hinzu, filtrierte und trocknete die abgetrennte organische Phase über Na₂SO₄. Eindampfen des Lösungsmittels im Wasserstrahlpumpenvakuum und Trocknung des erhaltenen Rückstandes im Ölpumpenvakuum lieferte 18,02 g *N*-[(9*H*-Fluoren-9-ylmethoxy)-carbonyl]-hydrazin (70,8 mmol) als farblosen Feststoff vom Schmelzpunkt 150-153 °C.
   **¹H-NMR** (250 MHz, DMSO-d₆, 300 K) δ = 10.10 (bs, 1H, N*H*), 9.60 (bs, 1H, N*H*), 7.89 (d, *J* = 7.6 Hz, 2H, arom), 7.70 (d, *J* = 7.3 Hz, 2H, arom), 7.30-7.45 (m, 4H, arom), 4.48 (d, *J* = 6.6 Hz, 2H, CO-C*H*₂), 4.27 (t, *J* = 6.7 Hz, 1H, CO-CH₂-C*H*).
B) Eine Suspension aus 1,49 g des vorstehend erhaltenen *N-*[(9*H*-fluoren-9-ylmethoxy)-carbonyl]-hydrazins (5,78 mmol), 60 ml Dichlormethan und 60 ml gesättigter wässriger NaHCO₃-Lösung wurde 5 Min. lang bei 0 °C stark gerührt und anschließend 5 Min. lang bei dieser Temperatur stehengelassen. Dann fügte man mit einer Spritze 7,95 ml einer 1,89 M Phosgen-Lösung in Toluol zu der unteren organischen Phase hinzu. Nach vollständiger Zugabe wurde das Reaktionsgemisch weitere 10 Min. lang heftig gerührt. Danach gab man zu der Reaktionsmischung 20 ml Wasser und 20 ml Dichlormethan hinzu und trennte die Phasen rasch. Die wässrige Phase wurde mit 50 ml Dichlormethan extrahiert und die vereinigten organischen Phasen wurden über Na₂SO₄ getrocknet. Nach Abdampfen des Lösungsmittels im Wasserstrahlpumpenvakuum und Trocknen des Rückstandes im Ölpumpenvakuum erhielt man 1,35 g 5-(9*H*-Fluoren-9-ylmethoxy)-3*H*-[1,3,4]oxadiazol-2-on (4,82 mmol) als farblosen Feststoff vom Schmelzpunkt 125 °C.
   **¹H-NMR** (250 MHz, CDCl₃ 300 K) δ = 8.72 (bs, 1H, N*H*), 7.77 (d, *J* = 7.5 Hz, 2H, arom), 7.59 (d, *J* = 7.4 Hz, 2H, arom), 7.28-7.45 (m, 4H, arom), 4.49 (d, J = 7.8 Hz, 2H, C*H*₂-CH), 4. 32-4.41 (m, 1H, CH₂-C*H*).
C) 100 mg eines mit Fmoc-D-Trp(Boc)-Phenylethylamid beladenen FMPE-Harzes (Herstellung siehe Beispiel 1A); Beladung 0,354 mmol/g; entsprechend 22,3 mg (0,035 mmol) freien Fmoc-D-Trp(Boc)-Phenylethylamids) ließ man 10 Min. lang in 5 ml NMP quellen und behandelte es dann zweimal je 15 Min. lang mit je 5 ml einer frisch bereiteten 20%igen (v/v) Lösung von Piperidin in NMP. Im Anschluss wusch man das Harz fünfmal je drei Min. lang mit jeweils 5 ml NMP und nochmals fünfmal je drei Min. lang mit jeweils 5 ml Dichlormethan. Dann ließ man das Harz 30 Min. lang in 5 ml trockenem Dichlormethan stehen. Nach Abtrennung des Lösungsmittels durch Filtration versetzte man das mit D-Trp(Boc)-Phenylethylamid beladene FMPE-Harz mit einer Lösung von 30,5 mg des vorstehend unter B) erhaltenen 5-(9*H*-fluoren-9-ylmethoxy)-3*H*-[13,4]oxadiazol-2-ons in 1 ml trockenem Dichlormethan und schüttelte die Mischung 90 Min. lang. Zuletzt wurde das Harz fünfmal drei Minuten lang mit je 5 ml Dichlormethan und anschließend nochmals fünfmal drei Min. lang mit je 5 ml NMP gewaschen. Man erhielt ein mit Fmoc-Hydrazin-carbonyl-D-Trp(Boc)-Phenylethylamid beladenes FMPE-Harz, welches ohne Isolierung des Zwischenproduktes direkt für die nachfolgend angegebene Umsetzung eingesetzt wurde.
D) Die gesamte Menge des vorstehend erhaltenen beladenen FMPE-Harzes [bei angenommener 100%iger Umsetzung beladen mit 0,354 mmol/g Fmoc-Hydrazin-carbonyl-D-Trp(Boc)-Phenylethylamid, entsprechend 24 mg (0,035 mmol) der freien Verbindung] wurde zur Abspaltung der Fmoc-Schutzgruppe wie in Beispiel 1B) beschrieben behandelt. Man erhielt ein mit Hydrazin-carbonyl-D-Trp(Boc)-Phenylethylamid beladenes FMPE-Harz, welches ohne Abspaltung und Isolierung des Zwischenproduktes direkt für die nachfolgend angegebene Umsetzung eingesetzt wurde.
E) Die gesamte Menge des vorstehend erhaltenen beladenen FMPE-Harzes [bei angenommener 100%iger Umsetzung beladen mit 0,354 mmol/g Hydrazin-carbonyl-D-Trp(Boc)-Phenylethylamid, entsprechend 16,5 mg (0,035 mmol) der freien Verbindung] wusch man fünfmal drei Min. lang mit je 5 ml NMP. Anschließend gab man zu dem beladenen FMPE-Harz eine Lösung von 12 mg 3-Pyridylessigsäure (0,07 mmol), 9,5 mg HOBT×H₂O (0,07 mmol) und 22,5 mg TBTU (0,07 mmol) in 2 ml NMP hinzu. Schließlich fügte man zu der entstandenen Vorlage 34 µl DIPEA (0,2 mmol) zu und schüttelte 45 Min. lang. Nachdem das FMPE-Harz fünfmal drei Min. lang mit je 5 ml NMP gewaschen worden war, wiederholte man den vorstehend beschriebenen Kopplungsschritt. Abschließend wusch man nochmals fünfmal drei Min. lang mit je 5 ml NMP. Man erhielt ein mit 3-Pyridylacetat-hydrazin-carbonyl-D-Trp(Boc)-Phenylethylamid beladenes FMPE-Harz, welches ohne Isolierung des Zwischenproduktes direkt für die nachfolgend angegebene Umsetzung eingesetzt wurde.
F) Die gesamte Menge des vorstehend erhaltenen beladenen FMPE-Harzes [bei angenommener 100%iger Umsetzung beladen mit 0,354 mmol/g 3-Pyridylacetat-Hydrazin-carbonyl-D-Trp(Boc)-Phenylethylamids, entsprechend 20,5 mg (0,035 mmol) der freien Verbindung] wurden zur Abspaltung vom FMPE-Harz und Entfernung der Boc-Schutzgruppe wie unter Beispiel 1G) beschrieben behandelt. Nach HPLC-Reinigung und Lyophilisierung erhielt man 4,5 mg (0,0093 mmol) der Titelverbindung als farbloses Pulver vom Schmelzpunkt 116-120 °C.
   **¹H-NMR** (500 MHz, DMSO-d₆, 300 K) δ= 10.79 (s, 1H, N*H*), 9.89 (s, 1H, N*H*), 8.63 (s, 1H, arom), 8.61 (d, *J* = 5.0 Hz, 1H, arom), 8.02-8.04 (m, 2H, N*H* and arom), 7.96 (bs, 1H, N*H*-CH₂-CH₂), 7.63 (t, *J* = 5.5 Hz, 1H, arom), 7.51 (d, *J* = 7.9 Hz, 1H, arom), 7.30 (d, *J* = 8.2 Hz, 1H, arom), 7.25 (t, *J* = 7.6 Hz, 2H, arom), 6.99-7.18 (m, 5H, arom), 6.95 (t, *J* = 8.0 Hz, 1H, arom), 6.44 (d, *J* = 8.1 Hz, 1H, N*H-*CH), 4.32-4.36 (m, 1H, NH-C*H*), 3.59 (s, 2H, CO-C*H*₂-C₅H₄N) 3.22-3.26 (m, 1H, NH-C*H*₂-CH₂), 3.15-3.19 (m, 1H, NH-C*H*₂-CH₂), 3.01 (dd, *J* = 14.4 Hz, *J* = 5.6 Hz, 1H, NH-CH-C*H*₂), 2.91 (dd, *J* = 14.6 Hz, *J* = 7.4 Hz, 1H, NH-CH-C*H*₂), 2.58 (t , *J* = 7. 5 Hz, 2H, NH-CH₂-C*H*₂).
   **HPLC-MS** (ESI) m/z 152.1 (40), 185.2 (30), 334.3 (30), 485.3 (100) [M+H]⁺, 507.3 (70) [M+Na]⁺, 523.3 (10) [M+Na]⁺, 969.3 (20) [2M+H]⁺, 991.4 (50) [2M+Na]⁺, 1007.5 (20) [2M+K]⁺.

### Beispiel 5:

### N1-Phenethyl-(2R)-2-[N'-(4-chlorbenzyl)-hydrazino]-carboxamido-3-(1H-3-indolyl)-propanamid (185)

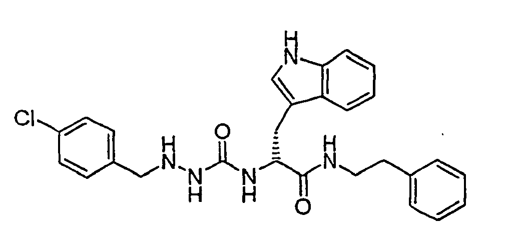

A) Zu einer Lösung von 1,98 g *tert*-Butylcarbazat (Boc-Hydrazin) in 15 ml THF tropfte man unter ständigem Rühren bei Raumtemperatur über einen Zeitraum von 10 Min. 2,12 g 4-Chlorbenzaldehyd in 5 ml THF hinzu. Nach 3 h wurde das Lösungsmittel im Wasserstrahlpumpenvakuum eingedampft und der erhaltene Rückstand wurde mittels Flash-Chromatographie unter einem Druck von 1-1,2 bar (stationäre Phase: Silicagel 60, Korngröße 0,040-0,063 mm, mobile Phase: Essigsäureethylester/Hexan 1:5) gereinigt. Nach Eindampfen des Lösungsmittels im Wasserstrahlpumpenvakuum und Trocknen des Rückstandes im Ölpumpenvakuum erhielt man 3,64 g *N*'-(4-Chlorphenylmethylen)-hydrazin-carbonsäure-*tert-*butylester als farblosen Feststoff vom Schmelzpunkt 170-171°C.
   **MS** (EI) m/z 41.2 (20), 57.2 (100), 154.0 (10), 181.0 (5), 197.9 (20), 253.9 (5) [M]⁺.
B) Zu einer Suspension von 1,5 g des vorstehend erhaltenen *N*'-(4-Chlorphenylmethylen)-hydrazincarbonsäure-*tert*-butyl-esters in 25 ml trockenem THF fügte man unter Eiskühlung und unter Argon-Schutzgasatmosphäre 0,55 g NaCNBH₃ hinzu. Zu dieser Mischung tropfte man über einen Zeitraum von 10 Min. 10 ml Essigsäure hinzu. Die entstandene klare Lösung ließ man über Nacht bei RT rühren. Dann fügte man 60 ml Wasser und 60 ml Essigsäureethylester hinzu und stellte den pH Wert der wässrigen Phase mit NaHCO₃ auf 8 ein. Die organische Phase wurde abgetrennt und der Reihe nach mit 50 ml gesättigter wässriger NaHCO₃-Lösung und mit 50 ml gesättigter wässriger Kochsalzlösung gewaschen. Man trocknete die organische Phase über Na₂SO₄ und dampfte das Lösungsmittel im Wasserstrahlpumpenvakuum ein. Der verbliebene farblose Rückstand wurde der Reihe nach mit 40 ml Methanol und mit 20 ml 1N NaOH versetzt und die entstandene Mischung wurde zunächst 1 h lang bei RT gerührt und dann 1 h lang unter Rückflusskühlung zum Sieden erhitzt. Man extrahierte die auf RT abgekühlte Mischung dreimal mit Diethylether, trocknete die vereinigten Etherphasen über Na₂SO₄ und dampfte das Lösungsmittel im Wasserstrahlvakuum ein. Das verbliebene gelbe Öl wurde mittels Flash-Chromatographie unter einem Druck von 1-1,2 bar (stationäre Phase: Silicagel 60, Korngröße 0,040-0,063 mm, mobile Phase: Essigsäureethylester/Hexan 1:4) gereinigt. Eindampfen des Lösungsmittels im Wasserstrahlpumpenvakuum und Trocknen des Rückstandes im Ölpumpenvakuum lieferte 1,05 g *N*-(*tert*-Butoxycarbonyl)-*N*'-(4-chlorbenzyl)-hydrazin als farblosen Feststoff vom Schmelzpunkt 77-82 °C.
   **¹H-NMR** (250 MHz, DMSO-d₆, 300 K) δ = 8.23 (bs, 1H, N*H*-CO), 7.35 (m, 4H, arom), 4.84 (bs, 1H, N*H*-CH₂), 3.85 (s, 2H, NH-C*H*₂), 1.37 (s, 9H, C*H*₃) .
C) 0,8 g des vorstehend erhaltenen *N*-(*tert*-Butoxycarbonyl)-*N*'-(4-chlorbenzyl)-hydrazins wurden unter Eiskühlung in einer Mischung aus 4 ml Dioxan und 16 ml 10%iger wässriger NaHCO₃-Lösung suspendiert. Dann fügte man über einen Zeitraum von 10 Min. eine Lösung von 0,89 g FmocCl in 10 ml Dioxan hinzu und ließ das Reaktionsgemisch anschließend über Nacht bei RT rühren. Man gab 50 ml Wasser hinzu und extrahierte die wässrige Phase dreimal mit je 100 ml Diethylether. Die abgetrennten organischen Phasen wurden vereinigt, über Na₂SO₄ getrocknet und das Lösungsmittel wurde schließlich im Wasserstrahlpumpenvakuum eingedampft. Der Rückstand wurde mittels Flash-Chromatographie unter einem Druck von 1-1,2 bar (stationäre Phase: Silicagel 60, Korngröße 0,040-0,063 mm, mobile Phase: Essigsäureethylester/Hexan 1:2) gereinigt. Eindampfen des Lösungsmittels im Wasserstrahlpumpenvakuum und Trocknen des Rückstandes im Ölpumpenvakuum lieferte 1,37 g *N*-(4-Chlorbenzyl)-*N-*[(9*H*-fluoren-9-ylmethoxy)-carbonyl]-*N*'-(*tert*-butoxycarbonyl)-hydrazin als farblosen Feststoff vom Schmelzpunkt 53-55 °C.
   **¹H-NMR** (250 MHz, DMSO-*d₆*, 300 K) δ = 9. 62 (s, 1H, N*H*), 7.89 (d, *J* = 7.3 Hz, 2H, arom), 7.74 (d, *J* = 6.7 Hz, 1H, arom), 7.56 (m, 1H, arom), 7.27-7.43 (m, 7H, arom), 6.99 (m, 1H, aröm), 4.2-5.52 (m, 5H, N-CH₂ and CO-C*H*₂-C*H*), 1.43 (s, 9H, C*H*₃).
D) Zu einer Lösung von 0,30 g des oben unter C) erhaltenen *N*-(4-Chlorbenzyl)-*N*-[(9*H*-fluoren-9-ylmethoxy)-carbonyl]-*N*'-(*tert*-butoxycarbonyl)-hydrazins in 2,5 ml Dichlormethan gab man 0,1 ml Triisopropylsilan und kühlte die Mischung auf. 0 °C ab. Dann tropfte man über einen Zeitraum von 5 Min. 2,5 ml TFA hinzu und ließ die Lösung anschließend 30 Min. lang rühren. Das Lösungsmittel wurde im Wasserstrahlvakuum unter Stickstoffkühlung eingedampft und der Rückstand wurde in einer Lösung von 77 mg DMAP (0,63 mmol) in 10 ml trockenem Dichlormethan wieder aufgenommen. Diese Mischung fügte man über einen Zeitraum von 20 Min. tropfenweise und unter Rühren zu einer Lösung von 0,25 g Dipentafluorphenylcarbonat (0,63 mmol) in 20 ml trockenem Dichlormethan hinzu. Nach vollständiger Zugabe gab man zu der so erhaltenen Vorlage unter Rühren eine Lösung von 0,26 g *tert*-Butyl-3-[(2*R*)-2-amino-2-(phenethylcarbamoyl)-ethyl]-1*H*-1-indolcarboxylat (Herstellung siehe Beispiel 3D)), 77 mg DMAP (0,63 mmol) und 10 ml trockenem Dichlormethan hinzu. Man ließ 30 Min. lang bei RT rühren, dampfte das Lösungsmittel im Wasserstrahlpumpenvakuum ein und nahm den Rückstand in 4 ml Dichlormethan auf. Nun gab man 0,1 ml Triisopropylsilan hinzu und kühlte auf 0°C ab. Dann tropfte man über einen Zeitraum von 5 Min. 4 ml TFA hinzu und ließ anschließend 30 Min. lang rühren. Das Lösungsmittel wurde im Ölpumpenvakuum entfernt und der getrocknete Rückstand wurde 30 Min. lang bei RT mit 10 ml einer 20%igen (v/v) Lösung von Piperidin in DMF versetzt. Man dampfte das Lösungsmittel im Wasserstrahlpumpenvakuum mit stickstoffgekühlter Vorlage ein, nahm den verbliebenen Rückstand in DMSO auf und reinigte diesen durch Reversed-Phase-HPLC [(HPLC-System *Amersham Pharmacia Biotech* Äkta Basic 100F; Pumpensystem P-900 und Detektor UV-900; Säule ODS-A C₁₈ von *Omnicrom YMC* (250 mm x 20 mm, 10 µm. Flußrate: 8 mL/min); Elution mit linearem Gradienten (30 Min.) von Wasser (Solvens A) in Acetonitril (Solvens B) und 0.1 % (v/v) TFA]. Lyophilisierung der gereinigten Fraktionen lieferte 26,8 mg der Titelverbindung als farbloses Pulver vom Schmelzpunkt 75-80 °C.
   **¹H-NMR** (500 MHz, ACN-*d₃*, 300 K) δ = 9.74 (bs, 1H, N*H*), 7.91 (d, *J* = 7.7 Hz, 1H, arom), 7.98 (d, *J* = 8.1 Hz, 1H, arom), 7.58-7.83 (m, 12H, arom), 6.99 (bs, 1H, N*H*-CH), 6.89 (bs, 1H, N*H*-CH₂-CH₂), 4.87 (q, *J* = 6.9 Hz, NH-C*H*), 4.34 (bs, 2H, NH-C*H*₂-C₆H₄Cl) , 3.87-3.94 (m, 1H, NH-C*H*₂-CH₂), 3.76-3.82 (m, 1H, NH-C*H*₂-CH₂), 3.66 (d, *J* = 6.2 Hz, 2H, NH-CH-C*H*₂), 3.17 (t, *J* = 7.3 Hz, 2H, NH-CH₂-C*H*₂).
   HPLC-MS (ESI) m/z 490.1 (70) [M+H]⁺, 512.3 (50) [m+Na]⁺, 754.8 (100), 978.9 (25) [2M+H]⁺, 1001.0 (90) [2M+Na]⁺, 1063.1 (20).

### Beispiel 6:

### N1-Phenethyl-(2R)-2-[N'-(furan-2-ylmethylen)-hydrazino]-carboxamido-3-(1H-3-indolyl)-propanamid (189)

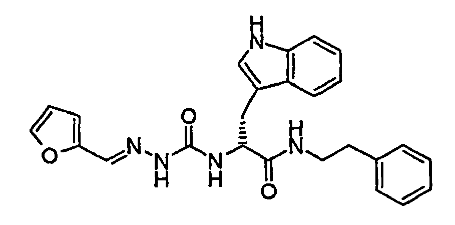

A) 500 mg *tert*-Butyl-3-[(2*R*)-2-amino-2-(phenethylcarbamoyl)-ethyl]-1*H*-1-indolcarboxylat (Herstellung siehe Beispiel 3D)) und 515 mg frisch hergestelltes 5-(9*H*-Fluoren-9-ylmethoxy)-3*H*-[1,3,4]oxadiazol-2-on (Herstellung siehe Beispiel 4B)) wurden in 20 ml trockenem DMF gelöst und 75 Min. lang bei RT gerührt. Anschließend wurde das Lösungsmittel im Wasserstrahlpumpenvakuum mit stickstoffgekühlter Vorlage eingedampft und der Rückstand wurde mittels Säulenchromatographie gereinigt (stationäre Phase: Silicagel 60, Korngröße 0,040-0,063 mm, mobile Phase:
   Chloroform/ Methanol, 20:1). Das Lösungsmittel wurde erneut im Wasserstrahlpumpenvakuum eingedampft und der Rückstand wurde im Ölpumpenvakuum getrocknet. Man erhielt 0,58 g *N*1-Phenethyl-(2*R*)-2-{[*N*'-(9*H*-fluoren-9-ylmethoxy)-carbonyl]-hydrazino}-carboxamido-3-[1-*tert*-butoxycarbonyl)-3-indolyl]-propanamid (= Fmoc-Hydrazin-carbonyl-D-Trp(Boc)-Phenylethylamid) als farblosen Feststoff vom Schmelzpunkt 135-137 °C.
   **HPLC-MS** (ESI) m/z 179.2 (10), 334.3 (10), 378.2 (10), 588.4 (10), 632.3 (25), 654.4 (35), 688.3 (80) [M+H]⁺, 710.4 (100) [M+Na]⁺, 1375.5 (40) [2M+H]⁺, 1397.5 (25) [2M+Na]⁺.
B) 179 mg des vorstehend erhaltenen Fmoc-Hydrazin-carbonyl-D-Trp(Boc)-Phenylethylamids wurden in 2 ml einer 20%igen (v/v) Lösung von Piperidin in DMF gelöst und 30 Min. lang bei RT gerührt. Dann wurde das Lösungsmittel im Wasserstrahlpumpenvakuum mit stickstoffgekühlter Vorlage eingedampft und der Rückstand wurde in 10 ml THF aufgenommen. Zu dieser Vorlage gab man 25 mg Furan-2-carbaldehyd hinzu und ließ 24 h lang bei RT rühren. Anschließendgab man weitere 50 mg Furan-2-carbaldehyd hinzu und ließ weitere 24 h lang rühren. Das Lösungsmittel wurde im Wasserstrahlpumpenvakuum eingedampft und der Rückstand wurde mittels Flash-Chromatographie unter einem Druck von 1-1,2 bar (stationäre Phase: Silicagel 60, Korngröße 0,040-0,063 mm, mobile Phase: Essigsäureethylester/Hexan 1:1) gereinigt. Nach Eindampfen des Lösungsmittels im Wasserstrahlpumpenvakuum und Trocknen des Rückstandes im Ölpumpenvakuum erhielt man 100 mg *N*1-Phenethyl-(2*R*)-2-[*N*'-(furan-2-ylmethylen)-hydrazino]-carboxamido-3-[1-(*tert*-butoxycarbonyl)-3-indolyl]-propanamid als farblosen, kristallinen Feststoff. **MS** (ESI) *m*/*z* 510.3 (15), 544.3 (55) [M+H]⁺, 566.3 (50) [M+Na]⁺, 835.2 (25) [(3M+K+H)/2]²⁺, 1087.4 (45) [2M+H]⁺, 1109.5 (100) [2M+Na]⁺, 1630.3 (5) [3M+H]⁺, 1652.2 (20) [3M+Na]⁺.
C) 100 mg des vorstehend erhaltenen *N*1-Phenethyl-(2*R*)-2-[*N*'-(furan-2-ylmethylen)-hydrazino]-carboxamido-3-[1-(*tert-*butoxycarbonyl)-3-indolyl]-propanamids wurden in 3 ml Dichlormethan gelöst. Hierzu fügte man 0,1 ml Triisopropylsilan und kühlte dann auf 0 °C ab. Dann tropfte man über einen Zeitraum von 5 Min. 3 ml TFA hinzu und ließ das Reaktionsgemisch 1 h lang rühren. Anschließend dampfte man das Lösungsmittel im Wasserstrahlpumpenvakuum ein, nahm den verbleibenden Rückstand in einer Mischung aus 8 ml DMSO, 1 ml Wasser und 1 ml Essigsäure auf und ließ über Nacht bei RT rühren. Dann wurde das Lösungsmittel im Wasserstrahlpumpenvakuum mit stickstoffgekühlter Vorlage bis zur Trockene eingedampft und der Rückstand wurde in DMSO aufgenommen. Reversed-Phase-HPLC [HPLC-System Amersham *Pharmacia Biotech* Äkta Basic 100F; Pumpensystem P-900 und Detektor UV-900; Säule ODS-A C₁₈ von *Omnicrom YMC* (250 mm X 20 mm, 10 µm. Flußrate: 8 mL/min; Elution mit linearem Gradienten (30 Min.) von Wasser (Solvens A) in Acetonitril (Solvens B) und 0.1 % (v/v) TFA] und Lyophilisierung der gereinigten Fraktionen lieferte 46 mg der Titelverbindung als farbloses Pulver vom Schmelzpunkt 100-101 °C. **¹H-NMR** (500 MHz, DMSO-d₆, 300 K) δ = 10.82 (s, 1H, N*H*-CH-C), 10.41 (s, 1H, N-N*H*), 8.09 (t, *J* = 5.5 Hz, 1H, N*H*-CH₂), 7.75 (s, 1H, arom), 7.73 (s, 1H, arom), 7.55 (d, *J* = 7.9 Hz, 1H, arom), 7.30 (d, *J* = 8.1 Hz, 1H, arom), 7.23-7.26 (m, 2H, arom), 7.14-7.17 (m, 3H, arom), 7.07 (s, 1H, a-rom), 7.03 (t, *J* = 7.4 Hz, 1H, arom), 6.93 (t, *J* = 7.5 Hz, 1H, arom), 6.74 (d, *J* = 3.2 Hz, 1H, arom), 6.58-6.60 (m, 2H, N*H*-CH-CH₂ and arom), 4.45 (q, *J* = 6.8 Hz, 1H, NH-C*H*), 3.24-3.31 (m, 1H, NH-C*H*₂), 3.17-3.24 (m, 1H, NH-C*H*₂), 3.02-3.10 (m, 2H, NH-CH-C*H*₂), 2.62 (t, *J* = 7.4 Hz, 2H, NH-CH₂-C*H*₂).
   HPLC-MS (ESI) m/z 444.2 (30) [M+H]⁺, 466.3 (65) [M+Na]⁺, 685.1 (90), 909.2 (100) [2M+Na]⁺, 1352.1 (15) [3M+Na]⁺.

### Beispiel 7 (nicht Teil der Erfindung):

### N1-Phenethyl-(2R)-2-[(4-benzylpiperidino)-methyl]-carbox-amido-3-(1H-3-indolyl)-propanamid

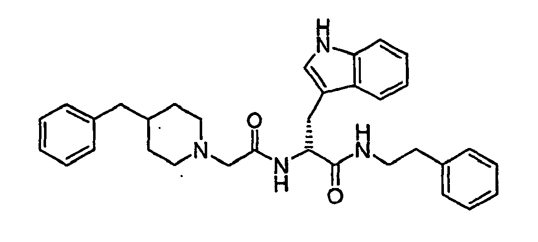

A) Zu 13 ml Toluol tropfte man unter Rühren und Eiskühlung 3,0 g 4-Benzylpiperidin und 1,73 g Triethylamin hinzu. Zu dieser Vorlage wurde über einen Zeitraum von 4 h tropfenweise eine Lösung von 2,86 g Bromessigsäureethylester in 6,2 ml Toluol zugegeben. Im Anschluss ließ man das Reaktionsgemisch 4 Tage lang bei RT rühren. Dann extrahierte man die organische Phase mit 100 ml Wasser und trocknete sie anschließend über Na₂SO₄. Das Lösungsmittel wurde im Wasserstrahlpumpenvakuum eingedampft und der Rückstand wurde im Ölpumpenvakuum getrocknet. Man erhielt 4,02 g (4-Benzyl-piperidin-1-yl)-essigsäureethylester als farbloses Öl.
   EPLC-MS (ESI) *m*/*z* 188.2 (100), 234.2 (45), 262.2 (100) [M+H]⁺.
B) 1,0 g des vorstehend erhaltenen (4-Benzyl-piperidin-1-yl)-essigsäureethylesters wurden zu einer Vorlage aus 5,755 ml 1N wässriger NaOH und 11,5 ml Methanol gegeben. Man ließ über Nacht bei RT rühren, neutralisierte dann mit konz. Salzsäure und dampfte das Lösungsmittel im Wasserstrahlpumpenvakuum ein. Der Rückstand wurde unter einem Druck von 1-1,2 bar mittels Flash-Chromatographie (stationäre Phase: Silicagel 60, Korngröße 0,040-0,063 mm, mobile Phase: Methanol/Chloroform, 1:1) gereinigt. Nach Eindampfen des Lösungsmittels im Wasserstrahlpumpenvakuum und Trocknen des Rückstandes im Ölpumpenvakuum erhielt man 0,89 g (4-Benzyl-piperidin-1-yl)-essigsäure als farblosen Feststoff vom Schmelzpunkt 250-252 °C.
   GC-MS (EI) *m*/*z* 44.1 (10), 91.1 (15), 188.1 (100), 233.0 (5) [M]⁺.
C) 86 mg der vorstehend unter B) erhaltenen (4-Benzylpiperidin-1-yl)-essigsäure, 150 mg tert-Butyl-3-[(2R)-2-amino-2-(phenethylcarbamoyl)-ethyl]-1*H-*1-indol-carboxylat (Herstellung siehe Beispiel 3D)), 75 mg HOBT und 177 mg TBTU wurden in 2,6 ml DMF gelöst. Zu dieser Vorlage wurden über einen Zeitraum von 5 Min. 0,20 g DIPEA hinzugetropft. Dann ließ man das Reaktionsgemisch 23 h lang rühren, dampfte das Lösungsmittel im Wasserstrahlpumpenvakuum mit stickstoffgekühlter Vorlage ein und reinigte den verbliebenen Rückstand mittels Flash-Chromatographie unter einem Druck von 1-1,2 bar (stationäre Phase: Silicagel 60, Korngröße 0,040-0,063 mm, mobile Phase: Chloroform/Methanol, 10:1). Man erhielt 180 mg *N*1-Phenethyl-(2R)-2-[(-benzyl-piperidino)-methyl]-carboxamido-3-[1-(tert-butoxycarbonyl)-3-indolyl]-propanamid als gelbes Öl.
   **HPLC-MS** (ESI) *m*/*z* 188.1 (20), 567.3 (70), 623.3 (100) [M+H]⁺, 645.2 (25) [M+Na]⁺, 1245.2 (10) [2M+H]⁺, 1267.3 (40) [2M+Na]⁺.
D) 180 mg des vorstehend erhaltenen *N*1-Phenethyl-(2R)-2-[(4-benzylpiperidino)-methyl]-carboxamido-3-[1-(tert-butoxycarbonyl)-3-indolyl]-propanamids wurden in 3 ml Dichlormethan gelöst. Hierzu fügte man 0,1 ml Triisopropylsilan und kühlte die Lösung auf 0 °C ab. Dann tropfte man über einen Zeitraum von 5 Min. 3 ml TFA hinzu und ließ das Reaktionsgemisch 1 h lang rühren. Im Anschluss wurde das Lösungsmittel im Wasserstrahlpumpenvakuum mit stickstoffgekühlter Vorlage eingedampft. Der verbliebene Rückstand wurde in einer Mischung aus 8 ml DMSO, 1 ml Wasser und 1 ml Essigsäure aufgenommen und über Nacht gerührt. Dann wurde das Lösungsmittel im Wasserstrahlpumpenvakuum mit stickstoffgekühlter Vorlage bis zur Trockne eingedampft. Den Rückstand nahm man in DMSO auf und reinigte diesen durch Reversed-Phase-HPLC [HPLC-System von *Amersham Pharmacia Biotech* Äkta Basic 100F; Pumpensystem P-900 und Detektor UV-900; Säule ODS-A C₁₈ von *Omnicrom YMC* (250 mm × 30 mm, 10 µm, Flußrate: 25 mL/min; Elution mit linearem Gradienten (30 Min.) von Wasser (Solvens A) in Acetonitril (Solvens B) und 0.1 % (v/v) TFA]. Lyophilisierung der gereinigten Fraktionen lieferte 109 mg der Titelverbindung als farbloses Pulver vom Schmelzpunkt 73-75 °C.
   **¹H-NMR** (500 MHz, DMSO-d₆, 300 K) δ = 10.84 (s, 1H, N*H-*CH-C), 8.81 (d, *J* = 8.3 Hz, 1H, N*H-*CH), 8.25 (t, *J* = 5.2 Hz, N*H-*CH₂-CH₂), 7.61 (d, *J* = 7.7 Hz, 1H, arom), 7.07-7.33 (m, 12H, arom), 7.02 (t, *J* = 7.3 Hz, 1H, arom); 6.96 (t, *J* = 6.9 Hz, 1H, arom), 4.60 (q, *J* = 6.9 Hz, 1H, N*H-*CH), 3.81 (d, *J* = 15.2 Hz, 1H, N-C*H*₂-CO) , 3.67 (d, *J* = 13.1 Hz, 1H, N-C*H*₂-CO), 3.21-3.34 (m, 3H, NH*-*C*H*₂-CH₂ and N-CH₂-CH₂-CH), 3.06 (dd, *J* = 14.4 Hz, *J* = 4.9 Hz, 1H, NH-CH-C*H*₂), 2.97-2.93 (m, 3H, NH-CH-C*H*₂ and N-C*H*₂-CH₂-CH), 2.59-2.67 (m, 3H, NH*-*CH₂-C*H*₂ and N-C*H*₂-CH₂-CH), 2.46-2.48 (m, 2H, CH-C*H*₂-C₆H₅) , 1.57-1.70 (m, 3H, N-CH₂-C*H*₂-CH and C*H*) , 1.32-1.46 (m, 2H, NCH₂-C*H*₂-CH).
   **HPLC-MS** (ESI) *m*/*z* 188.1 (70), 523.3 (100) [M+H]⁺, 803.7 (20), 1045.1 (20) [2M+H]⁺, 1067.3 (40) [2M+Na]⁺.

Nach den vorstehend angegebenen Herstellungsverfahren oder analog zu diesen Herstellungsverfahren können auch die nachfolgend in Tabelle 2 angegebenen Verbindungen der Formel I hergestellt werden. Tabelle 2 enthält folgende Abkürzungen:

| | |
|---|---|
| bi: | Bindung |
| dm: | dioxolanylmethyl |
| Ind: | Indolyl |
| Phe: | Phenyl |
| Py: | Pyridyl |
| rac.: | racemisch |
| THI: | Tetrahydroisochinolyl |

**Tabelle 2: Weitere Verbindungen der Formel I**

| **Bsp.** Nr | **A1** | **A²** | **A³** | **R¹** | **R²** | **R³** | **Ar¹** | **Ar²** | **Ar³** | **m** | **n** | **(HR)MS m/z** |
|---|---|---|---|---|---|---|---|---|---|---|---|---|
| 8 | (R) -CH- | C(O) | (S)-CH-[(CH₂)₂(CO)NH₂] | -NH₂ | H | H | Phe | 3-Ind | Phe | 1 | 0 | |
| 9 | (R) -CH- | C(O) | (S)-CH-[(CH₂)₂(CO)NH₂] | -NH₂ | H | H | Phe | 3-Ind | 4-Br-Phe | 1 | 1 | |
| 10 | (R) -CH- | C(O) | (S)-CH-(CH₂)₂ (CO) NH₂] | -NH₂ | H | H | Phe | 3-Ind | 2-Py | 1 | 1 | |
| 11 | (R) -CH- | C(O) | (S)-CH-[(CH₂)₂(CO)NH₂] | -NH₂ | H | (R) -CH₃ | Phe | 3-Ind | Phe | 1 | 1 | |
| 12 | (R) -CH- | C(O) | (S)-CH-[(CH₂)₂(CO)NH₂] | -NH₂ | H | (S) -CH₃ | Phe | 3-Ind | Phe | 1 | 1 | |
| 13 | -CH- | C(O) | (S)-CH-[(CH₂)₂ (CO) NH₂] | H | H | H | 1-THI (rac) | 3-Ind | Phe | 0 | 1 | 609,32 |
| 14 | -CH- | C(O) | (S)-CH-[(CH₂)₂(CO)NH₂] | H | H | H | 4-C1-Phe | 3-Ind | Phe | 1 | 1 | 602,25 |
| 15 | -CH- | C(O) | (S)-CH-[ (CH₂)₂ (CO) NH₂] | H | H | H | 2-Ind | 3-Ind | Phe | 0 | 1 | 593,29 |
| 16 | -CH- | C(O) | (S)-CHCH₃ | H | H | H | 4-Cl-Phe | 3-Ind | Phe | 0 | 1 | 531,21 |
| 17 | -CH- | C(O) | (S)-CHCH₃ | H | H | H | 1-THI (rac) | 3-Ind | The | 0 | 1 | 552,30 |
| 18 | -CH- | C(O) | (S)-CHCH₃ | H | H | H | 3,4-dm-Phe | 3-Ind | Phe | 0 | 1 | 541,25 |
| 19 | -CH- | C(O) | (S)-CHCH₃ | H | H | H | 3-Py | 3-Ind | Phe | 0 | 1 | 498,25 |
| 20 | -CH- | C(O) | (S) -CHCH₃ | H | H | H | 2-Ind | 3-Ind | Phe | 0 | 1 | 536,27 |
| 21 | -CH- | Bi | -CH₂ | H | H | H | Phe | 3-Ind | Phe | 0 | 1 | 455,24 |
| 22 | -CH- | Bi | -CH₂ | H | H | H | 4-Cl-Phe | 3-Ind | Phe | 0 | 1 | 489,21 |
| 23 | -CH- | Bi | -CH₂ | H | H | H | 3-Py | 3-Ind | Phe | 0 | 1 | 456,24 |
| 24 | -CH- | Bi | -CH₂ | H | H | H | Phe | 3-Ind | Phe | 1 | 1 | 469,26 |
| 25 | -CH- | Bi | (S)-CHCH₃ | H | H | H | Phe | 3-Ind | Phe | 0 | 1 | 469,26 |
| 26 | -CH- | Bi | (S)-CHCH₃ | H | H | H | Phe | 3-Ind | Phe | 1 | 1 | 483,28 |
| 27 | -CH- | C(O) | -NH- | H | H | H | Phe | 3-Ind | Phe | 0 | 1 | 484,23 |
| 28 | -CH- | C(O) | -NH- | H | H | H | 4-Cl-Phe | 3-Ind | Phe | 0 | 1 | 518,19 |
| 29 | -CH- | C(O) | -NH- | H | H | H | 1-THI (rac.) | 3-Ind | Phe | 0 | 1 | 539,28 |
| 30 | -CH- | C(O) | -NH- | H | H | H | 2-Ind | 3-Ind | Phe | 0 | 1 | 523,24 |
| 31 | -CH- | Bi | -NH- | H | H | H | Phe | 3-Ind | Phe | 0 | 1 | 456,24 |
| 32 | -CH- | Bi | -NH- | H | H | H | 2-Furyl | 3-Ind | Phe | 0 | 1 | 446,22 |
| 33 | -CH- | Bi | -NH- | Bi | | H | Phe | 3-Ind | Phe | 0 | 1 | 454,22 |
| 34* | -N- | -(CH₂)₂- | -CH₂- | (CH₂) ₂ | | H | Phe | 3-Ind | Phe | 1 | 1 | 524,30 |

| | | | | | | | | | | | | |
|---|---|---|---|---|---|---|---|---|---|---|---|---|
| *Vergleichsbeispiel | | | | | | | | | | | | |

### Beispiel I:

### N1-Phenethyl-(2R)-2-{1-[(4-chlorbenzyl)-amino]-ethylcarbox-amido}-3-(1H-3-indolyl)-propanamid enthaltende Kapseln:

| Es werden Kapseln in folgender Zusammensetzung pro Kapsel hergestellt: | |
|---|---|
| *N*1-Phenethyl-(2*R*)-2-{1-[(4-chlorbenzyl)-amino] - | |
| ethylcarboxamido}-3-(1*H*-3-indolyl)-propanamid | 20 mg |
| Maisstärke | 60 mg |
| Milchzucker | 300 mg |
| Ethylacetat | q.s. |

Der Wirkstoff, die Maisstärke und der Milchzucker werden unter Zuhilfenahme von Ethylacetat zu einer homogenen pastösen Mischung verarbeitet. Die Paste wird zerkleinert und das entstehende Granulat wird auf ein geeignetes Blech gebracht und zur Entfernung des Lösungsmittels bei 45 °C getrocknet. Das getrocknete Granulat wird durch eine Zerkleinerungsmaschine geleitet und in einem Mixer mit weiteren folgenden Hilfsstoffen vermischt:

| | |
|---|---|
| Talkuum | 5 mg |
| Magnesiumstearat | 5 mg |
| Maisstärke | 9 mg |

und sodann in 400 mg fassende Kapseln (= Kapselgröße 0) abgefüllt.

## Patentansprüche

1. Verbindungen der allgemeinen Formel I, 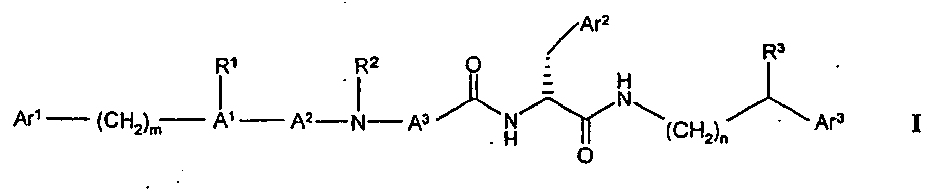 worin
A¹ CH oder, sofern nicht A² für eine Bindung und gleichzei-tig A³ für NH steht, auch Stickstoff bedeutet,
A² eine Bindung, C₁₋₂-Alkylen oder, sofern A¹ für CH steht und R² für Wasserstoff steht, auch Carbonyl bedeutet,
A³ Methylen, welches gegebenenfalls durch C₁₋₄-Alkyl oder C₁₋₄-Alkylcarbonylamid substituiert ist, oder, sofern R² Wasserstoff bedeutet oder gemeinsam mit R¹ für eine Bin-dung steht, auch NH bedeutet,
R¹ Wasserstoff oder, sofern A² für Carbonyl steht, auch Amino bedeutet und
R² Wasserstoff bedeutet, oder
R¹ und R² gemeinsam für eine Bindung stehen, sofern A² eine Bindung bedeutet
R³ Wasserstoff oder Methyl bedeutet,
Ar¹ Phenyl, welches gegebenenfalls 1- bis 2-fach durch Halo-gen oder C₁₋₄-Alkyl oder durch an zwei benachbarte Ring-kohlenstoffatome gebundenes C₁₋₂-Alkylendioxy substitu-iert ist, Pyridyl, Furyl, Indolyl oder Tetrahydroisochi-nolyl bedeutet,
Ar² Furyl, Benzofuranyl, Thienyl, Benzothiophenyl, Pyrrolyl oder Indolyl bedeutet,
Ar³ Phenyl, welches gegebenenfalls 1 bis 2-fach durch Halo-gen substituiert ist, oder Pyridyl bedeutet,
m 0 oder 1 bedeutet und
n 0 oder 1 bedeutet
sowie gegebenenfalls deren physiologisch verträgliche Säureadditionssalze.

2. Verbindungen der Formel I nach Anspruch 1, worin n für 1 steht und R³ Wasserstoff bedeutet.

3. Verbindungen der Formel I nach einem der vorstehenden Ansprüche, worin Ar² für Indolyl oder für Benzothiophenyl steht.

4. Verbindungen nach Anspruch 1 der allgemeinen Formel Ia, 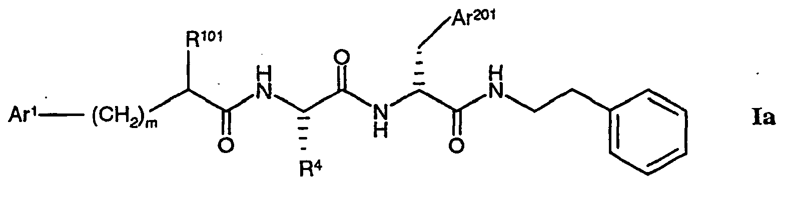 worin
R¹⁰¹ Wasserstoff oder Amino bedeutet,
R⁴ Wasserstoff, C₁₋₄-Alkyl oder C₁₋₄-Alkylcarbonylamid bedeutet,
Ar¹ Phenyl, welches gegebenenfalls 1- bis 2-fach durch Halogen oder C₁₋₄-Alkyl oder durch an zwei benachbarte Ringkohlenstoffatome gebundenes C₁₋₂-Alkylendioxy substituiert ist, Pyridyl, Furyl, Indolyl oder Tetrahydroisochinolyl bedeutet,
Ar²⁰¹ Benzothiophenyl oder Indolyl bedeutet und
m 0 oder 1 bedeutet
sowie gegebenenfalls deren physiologisch verträgliche Säureadditionssalze.

5. Verbindungen nach Anspruch 1 der allgemeinen Formel Ib, 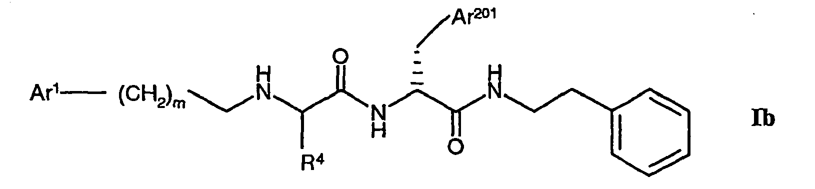 worin
R⁴ Wasserstoff, C₁₋₄-Alkyl oder C₁₋₄-Alkylcarbonylamid bedeutet,
Ar¹ Phenyl, welches gegebenenfalls 1- bis 2-fach durch Halogen oder C₁₋₄-Alkyl oder durch an zwei benachbarte Ringkohlenstoffatome gebundenes C₁₋₂-Alkylendioxy substituiert ist, Pyridyl, Furyl, Indolyl oder Tetrahydroisochinolyl bedeutet,
Ar²⁰¹ Benzothiophenyl oder Indolyl bedeutet und
m 0 oder 1 bedeutet
sowie gegebenenfalls deren physiologisch verträgliche Säureadditionssalze.

6. Verbindungen nach Anspruch 1 der allgemeinen Formel Ic, 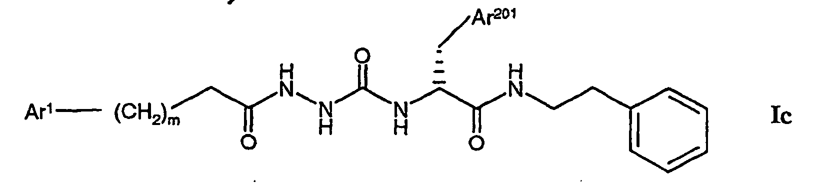 worin
Ar¹ Phenyl, welches gegebenenfalls 1- bis 2-fach durch Halogen oder C₁₋₄-Alkyl oder durch an zwei benachbarte Ringkohlenstoffatome gebundenes C₁₋₂-Alkylendioxy substituiert ist, Pyridyl, Furyl, Indolyl oder Tetrahydroisöchinolyl bedeutet,
Ar²⁰¹ Benzothiophenyl oder Indolyl bedeutet und
m 0 oder 1 bedeutet
sowie gegebenenfalls deren physiologisch verträgliche Säureadditionssalze.

7. Verbindungen nach Anspruch 1 der allgemeinen Formel Id, 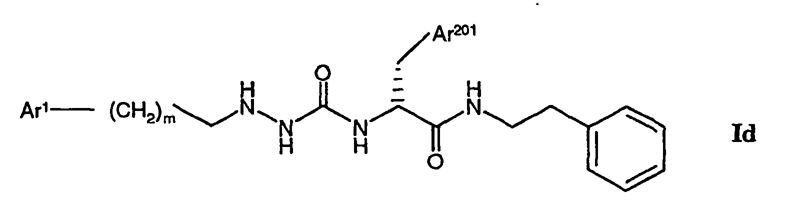 worin
Ar¹ Phenyl, welches gegebenenfalls 1- bis 2-fach durch Halogen oder C₁₋₄-Alkyl oder durch an zwei benachbarte Ringkohlenstoffatome gebundenes C₁₋₂-Alkylendioxy substituiert ist, Pyridyl, Furyl, Indolyl oder Tetrahydroisochinolyl bedeutet,
Ar²⁰¹ Benzothiophenyl der Indolyl bedeutet und
m 0 oder 1 bedeutet
sowie gegebenenfalls deren physiologisch verträgliche Säureadditionssalze.

8. Verbindungen nach Anspruch 1 der allgemeinen Formel Ie, 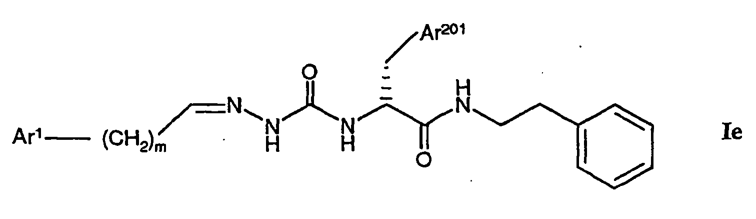 worin
Ar¹ Phenyl, welches gegebenenfalls 1- bis 2-fach durch Halogen oder C₁₋₄-Alkyl oder durch an zwei benachbarte Ringkohlenstoffatome gebundenes C₁₋₂-Alkylendioxy substituiert ist, Pyridyl, Furyl, Indolyl oder Tetrahydroisochinolyl bedeutet,
Ar²⁰¹ Benzothiophenyl oder Indolyl bedeutet und
m 0 oder 1 bedeutet
sowie gegebenenfalls deren physiologisch verträgliche Säureadditionssalze.

9. Verbindungen der Formel I nach Anspruch 1, zur Verwendung als Arzneimittels

10. Arzneimittel, enthaltend eine pharmakologisch wirksame Menge einer Verbindung der Formel I nach Anspruch I und zusätzlich übliche pharmazeutische Hilfs- und/oder Trägerstoffe.

11. Verwendung von Verbindungen der Formel I nach Anspruch 1 zur Herstellung eines Arzneimittels für die Behandlung und/oder Prophylaxe von Obesitas, Diabetes, Hyperinsulinämie, kardiovaskulären Erkrankungen, Essstörungen und/oder Syndrom X.

12. Verfahren zur Herstellung von Verbindungen der allgemeinen Formel I, 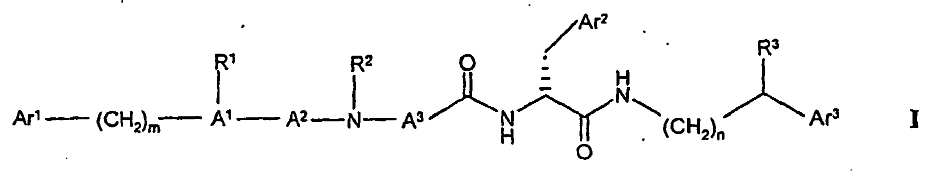 worin
A¹, A², A³, R¹, R², R³, Ar¹, Ar², Ar³, m, und n
die in Anspruch 1 genannte Bedeutung haben,
sowie gegebenenfalls deren physiologisch verträglicher Säureadditionssalze, **dadurch gekennzeichnet, daß** man
a) zur Herstellung einer Verbindung der allgemeinen Formel Ig, 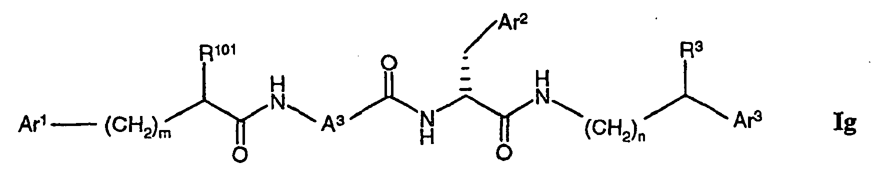 worin A³, R³, Ar¹, Ar², Ar³, m und n obige Bedeutungen besitzen und R¹⁰¹ Wasserstoff oder Amino bedeutet, eine Verbindung der allgemeinen Formel II, 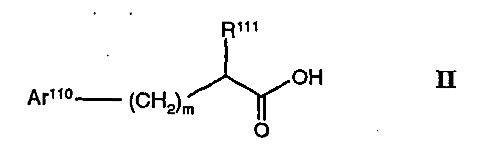 worin m obige Bedeutung besitzt, Ary¹¹⁰ die oben für Ar¹ angegebene Bedeutung besitzt, wobei allfällige reaktive Gruppen durch Schutzgruppen geschützt sind und R¹¹¹ die oben für R¹⁰¹ angegebene Bedeutung besitzt, wobei eine allfällige Aminogruppe durch eine Schutzgruppe geschützt ist, mit einer Verbindung der allgemeinen Formel III, 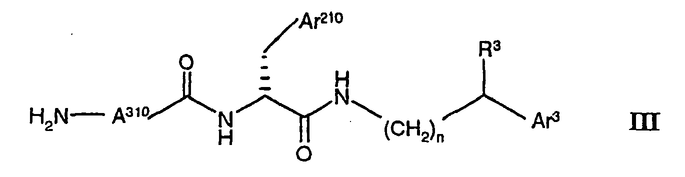 worin R^{3,} Ar³ und n obige Bedeutungen besitzen, Ar²¹⁰ die oben für Ar² angegebene Bedeutung besitzt, wobei allfällige reaktive Gruppen durch Schutzgruppen geschützt sind und A³¹⁰ die oben für A³ angegebene Bedeutung besitzt, wobei allfällige reaktive Stickstoffatome durch Schutzgruppen geschützt sind, umsetzt, oder
b) zur Herstellung einer Verbindung der allgemeinen Formel **Ih** 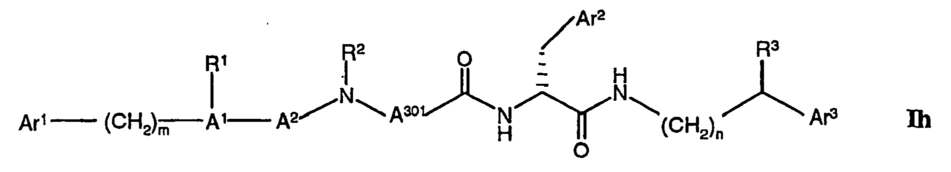 worin A¹, A² , R¹ , R² , R³ , Ar¹ , Ar² , Ar³ , m und n obige Bedeutungen besitzen und A³⁰¹ die oben für A³ angegebene Bedeutung mit Ausnahme von NH besitzt, eine Verbindung der allgemeinen Formel IV, 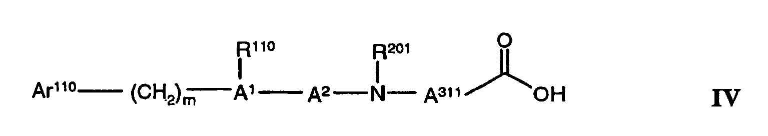 worin A¹, A², Ar¹¹⁰ und m obige Bedeutungen besitzen, A³¹¹ die oben für A³⁰¹ angegebene Bedeutung besitzt, wobei allfällige reaktive Stickstoffatome durch Schutzgruppen geschützt sind, R¹¹⁰ die oben für R¹ angegebene Bedeutung besitzt, wobei eine allfällige Aminogruppen durch eine Schutzgruppe geschützt ist und R²⁰¹ die oben für R² angegebene Bedeutung mit der Ausnahme von Wasserstoff besitzt, oder eine Aminoschutzgruppe bedeutet, mit einer Verbindung der allgemeinen Formel V, 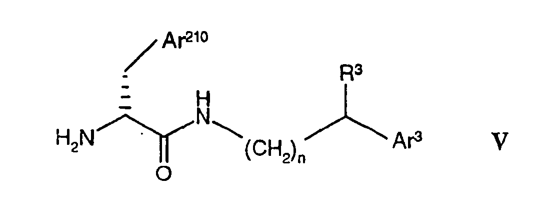 worin R³, Ar²¹⁰, Ar³ und n obige Bedeutungen besitzen, umsetzt, oder
c) zur Herstellung einer Verbindung der allgemeinen Formel **I**i, 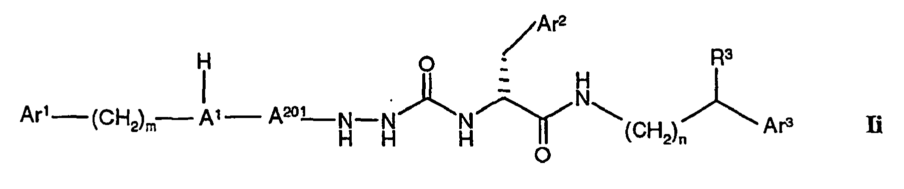 worin A¹, R³, Ar¹, Ar², Ar³, m und n obige Bedeutungen besitzen und A²⁰¹ die oben für A² angegebene Bedeutung mit Ausnahme von Carbonyl besitzt, eine Verbindung der Formel V mit einem Carbonylgruppen-Syntheseäquivalent und mit einer Verbindung der allgemeinen Formel VI, 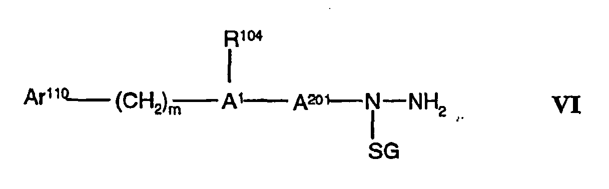 worin A¹, A²⁰¹, Ar¹¹⁰ und m obige Bedeutungen besitzen, R¹⁰⁴ für Wasserstoff oder, sofern A¹ Stickstoff bedeutet, auch für eine Stickstoffschutzgruppe stehen kann, und SG für eine in der Peptidchemie geeignete Schutzgruppe steht, umsetzt, oder
d) zur Herstellung einer Verbindung der allgemeinen Formel **Ij**, 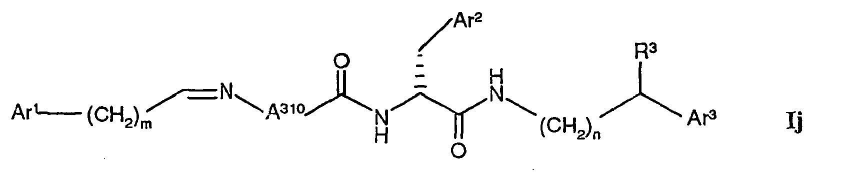
worin A³¹⁰, R³, Ar¹, Ar², Ar³, m und n obige Bedeutungen besitzen, eine Verbindung der Formel III mit einer Verbindung der allgemeinen Formel VIII, 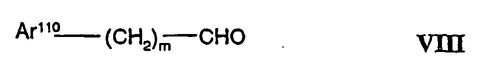 worin Ar¹¹⁰ und m obige Bedeutungen besitzen, umsetzt, und allfällige Schutzgruppen jeweils nachfolgend wieder abspaltet, und eine erhaltene Verbindung der Formel I gewünschtenfalls in ihr Säureadditionssalz überführt oder ein Säureadditionssalz in eine freie Verbindung der Formel I überführt.

## Claims

1. Compounds of the general formula **I**, 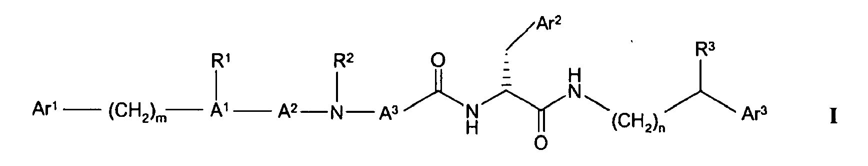 wherein
A¹ is CH or, if A² does not stand for a bond and at the same time A³ does not stand for NH, also nitrogen,
A² is a bond, C₁₋₂-alkylene or, if A¹ stands for CH and R² stands for hydrogen, also carbonyl,
A³ is methylene which is optionally substituted by C₁₋₄-alkyl or C₁₋₄-alkyl carbonylamide or, if R² is hydrogen or together with R¹ stands for a bond, is also NH,
R¹ is hydrogen or, if A² stands for carbonyl, also amino, and
R² is hydrogen, or
R¹ and R² together stand for a bond if A² is a bond,
R³ is hydrogen or methyl,
Ar¹ is phenyl which is optionally substituted 1 to 2 times by halogen or C₁₋₄-alkyl or by C₁₋₂-alkylenedioxy bonded to two adjacent ring carbon atoms; pyridyl, furyl, indolyl or tetrahydroisoquinolyl,
Ar¹ is furyl, benzofuranyl, thienyl, benzothiophenyl, pyrrolyl or indolyl,
Ar³ is phenyl which is optionally substituted 1 to 2 times by halogen, or pyridyl,
m is 0 or 1 and
n is 0 or 1,
and also optionally their physiologically compatible acid addition salts.

2. Compounds of Formula I according to Claim 1, wherein n stands for 1 and R³ is hydrogen.

3. Compounds of Formula I according to one of the preceding claims, wherein Ar² stands for indolyl or for benzothiophenyl.

4. Compounds according to Claim 1 of the general formula Ia, 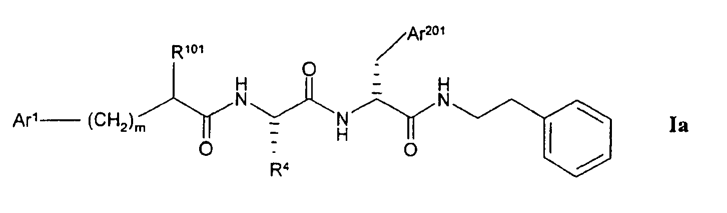 wherein
R¹⁰¹ is hydrogen or amino,
R⁴ is hydrogen, C₁₋₄-alkyl or C₁₋₄-alkyl carbonylamide,
Ar¹ is phenyl which is optionally substituted 1 to 2 times by halogen or C₁₋₄-alkyl or by C₁₋₂-alkylenedioxy bonded to two adjacent ring carbon atoms; pyridyl, furyl, indolyl or tetrahydroisoquinolyl,
Ar²⁰¹ is benzothiophenyl or indolyl and
m is 0 or 1
and also optionally their physiologically compatible acid addition salts.

5. Compounds according to Claim 1 of the general formula 1b, 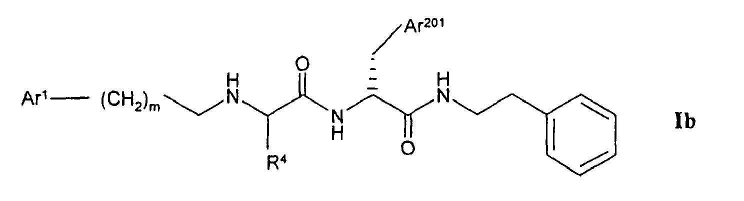 wherein
R⁴ is hydrogen, C₁₋₄-alkyl or C₁₋₄-alkyl carbonylamide,
Ar¹ is phenyl which is optionally substituted 1 to 2 times by halogen or C₁₋₄-alkyl or by C₁₋₂-alkylenedioxy bonded to two adjacent ring carbon atoms; pyridyl, furyl, indolyl or tetrahydroiso-quinolyl,
Ar²⁰¹ is benzothiophenyl or indolyl and
m is 0 or 1
and also optionally their physiologically compatible acid addition salts.

6. Compounds according to Claim 1 of the general formula Ic, 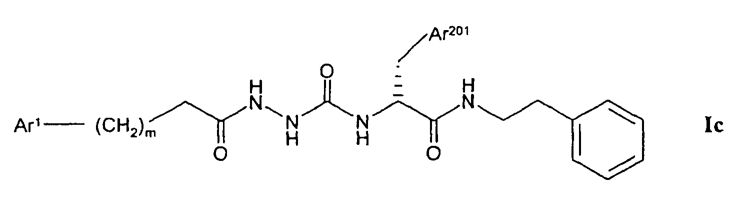 wherein
Ar¹ is phenyl which is optionally substituted 1 to 2 times by halogen or C₁₋₄-alkyl or by C₁₋₂-alkylenedioxy bonded to two adjacent ring carbon atoms, pyridyl, furyl, indolyl or tetrahydroisoquinolyl,
Ar²⁰¹ is benzothiophenyl or indolyl and
m is 0 or 1
and also optionally their physiologically compatible acid addition salts.

7. Compounds according to Claim 1 of the general formula Id, 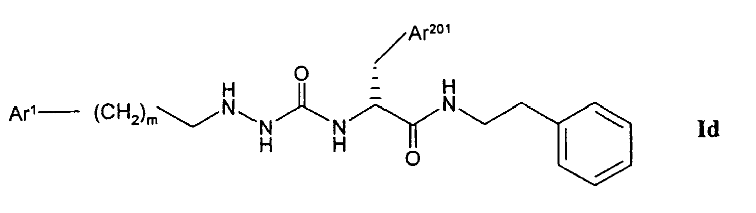 wherein
Ar¹ is phenyl which is optionally substituted 1 to 2 times by halogen or C₁₋₄-alkyl or by C₁₋₂-alkylenedioxy bonded to two adjacent ring carbon atoms, pyridyl, furyl, indolyl or tetrahydroisoquinolyl,
Ar²⁰¹ is benzothiophenyl or indolyl and
m is 0 or 1
and also optionally their physiologically compatible acid addition salts.

8. Compounds according to Claim 1 of the general formula Ie, 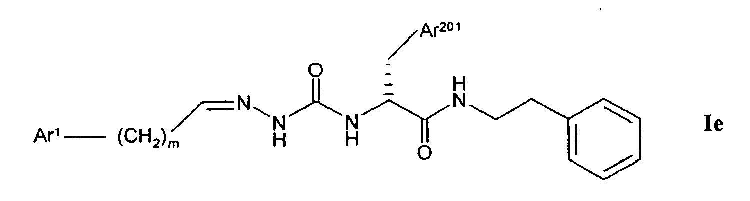 wherein
Ar¹ is phenyl which is optionally substituted 1 to 2 times by halogen or C₁₋₄-alkyl or by C₁₋₂-alkylenedioxy bonded to two adjacent ring carbon atoms, pyridyl, furyl, indolyl or tetrahydroisoquinolyl,
Ar²⁰¹ is benzothiophenyl or indolyl and
m is 0 or 1
and also optionally their physiologically compatible acid addition salts.

9. Compounds of Formula I according to Claim 1, for use as medicaments.

10. Medicament, containing a pharmacologically active quantity of a compound of Formula I according to Claim 1 and additionally conventional pharmaceutical auxiliaries and/or excipients.

11. The use of compounds of Formula I according to Claim 1 for the preparation of a medicament for the treatment and/or prophylaxis of obesity, diabetes, hyperinsulinism, cardiovascular diseases, eating disorders and/or syndrome X.

12. Process for the preparation of compounds of general formula I, 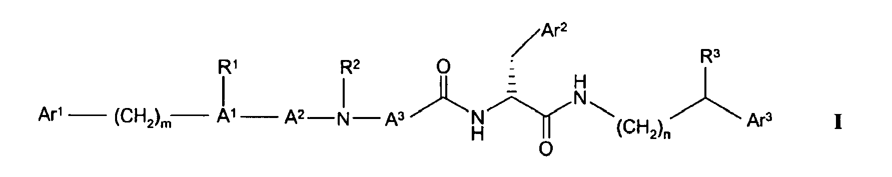 wherein
A¹, A², A³, R¹, R², R³, Ar¹, Ar², Ar³, m and n have the meaning mentioned in Claim 1, and also optionally their physiologically compatible acid addition salts,
**characterized in that**
a) for the preparation of a compound of the general formula Ig, 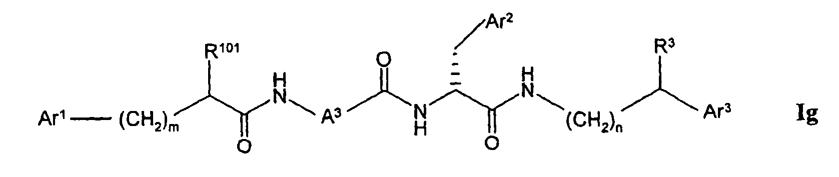 wherein A³, R³, Ar¹, Ar², Ar³, m and n have the above meanings, and R¹⁰¹ is hydrogen or amino, a compound of general Formula II, 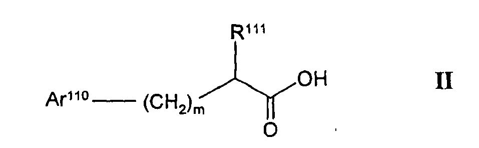 wherein m has the above meaning, Ar¹¹⁰ has the meaning given above for Ar¹, any reactive groups being protected by protective groups, and R¹¹¹ has the meaning given above for R¹⁰¹, any amino group being protected by a protective group, is reacted with a compound of the general formula III, 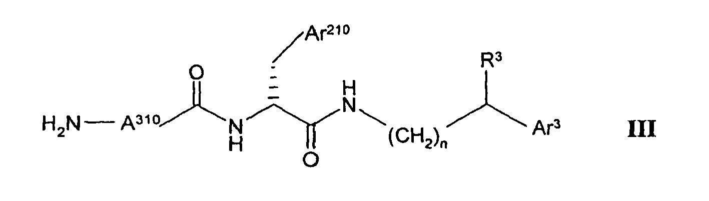 wherein R³, Ar³ and n have the above meanings, Ar²¹⁰ has the meaning given above for Ar², any reactive groups being protected by protective groups, and A³¹⁰ has the meaning given above for A³, any reactive nitrogen atoms being protected by protective groups, or
b) for the preparation of a compound of the general formula Ih 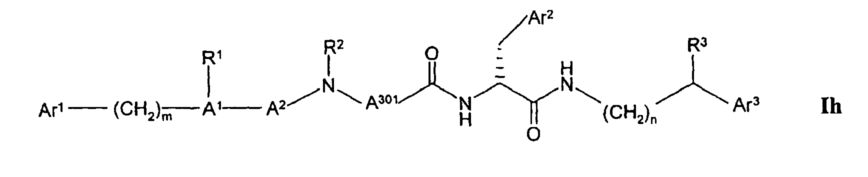 wherein A¹, A², R¹, R², R³, Ar¹, Ar², Ar³, m and n have the above meanings and A³⁰¹ has the meaning given above for A³ with the exception of NH, a compound of the general formula IV, 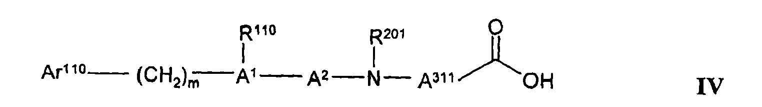 wherein A¹, A², Ar¹¹⁰ and m have the above meanings, A³¹¹ has the meaning given above for A³⁰¹, any reactive nitrogen atoms being protected by protective groups, R¹¹⁰ has the meaning given above for R¹, any amino groups being protected by a protective group, and R²⁰¹ has the meaning given above for R² with the exception of hydrogen, or represents an amino protective group, is reacted with a compound of the general formula V, 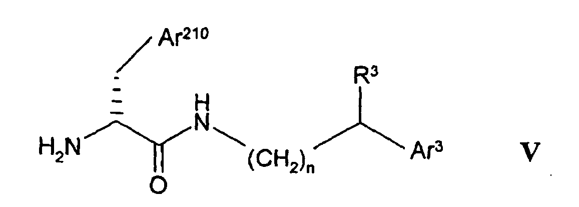 wherein R³, Ar²¹⁰, Ar³ and n have the above meanings, or
c) for the preparation of a compound of the general formula Ii, 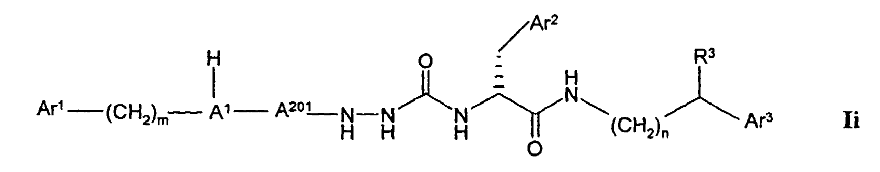 wherein A¹, R³, Ar¹, Ar², Ar³, m and n have the above meanings and A²⁰¹ has the meaning given above for A² with the exception of carbonyl, a compound of Formula V is reacted with a carbonyl-group synthesis equivalent and with a compound of the general formula VI, 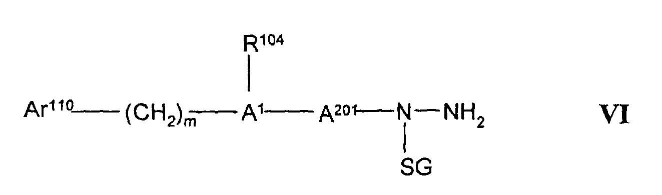 wherein A¹, A²⁰¹, Ar¹¹⁰ and m have the above meanings, R¹⁰⁴ stands for hydrogen or, if A¹ is nitrogen, may also stand for a nitrogen protective group, and SG stands for a protective group suitable in peptide chemistry, or
d) for the preparation of a compound of the general formula Ij, 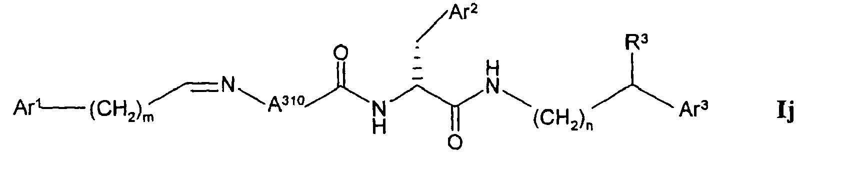
wherein A³¹⁰, R³, Ar¹, Ar², Ar³, m and n have the above meanings, a compound of Formula III is reacted with a compound of the general formula VIII, 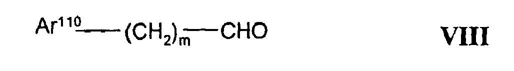 wherein Ar¹¹⁰ and m have the above meanings, and any protective groups are each subsequently cleaved off again, and a resulting compound of Formula I if desired is converted into its acid addition salt or an acid addition salt is converted into a free compound of Formula I.

## Revendications

1. Composés de formule générale I, 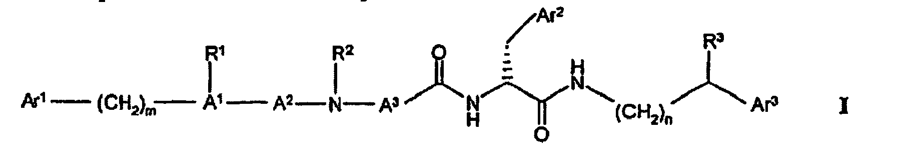 où
A¹ signifie CH ou, pour autant que A² ne représente pas une liaison et A³ ne représente pas simultanément NH, signifie également azote,
A² signifie une liaison, C₁₋₂-alkylène ou, pour autant que A¹ représente CH et R² représente hydrogène, signifie également carbonyle,
A³ signifie méthylène, qui est le cas échéant substitué par C₁₋₄-alkyle ou C₁₋₄-alkylcarbonylamide, ou, pour autant que R² signifie hydrogène ou, ensemble avec R¹, une liaison, signifie également NH,
R¹ signifie hydrogène ou, pour autant que A² représente carbonyle, signifie également amino et
R² signifie hydrogène, ou
R¹ et R² représentent ensemble une liaison, pour autant que A² représente une liaison
R³ signifie hydrogène ou méthyle,
Ar¹ signifie phényle, qui est le cas échéant monosubstitué ou disubstitué par halogène ou par C₁₋₄-alkyle ou par C₁₋₂-alkylènedioxy lié à deux atomes de carbone du cycle adjacents, pyridyle, furyle, indolyle ou tétrahydro-isoquinoléyle,
Ar² signifie furyle, benzofurannyle, thiényle, benzothiophényle, pyrrolyle ou indolyle,
Ar³ représente phényle, qui est le cas échéant monosubstitué à disubstitué par halogène, ou signifie pyridyle,
m signifie 0 ou 1 et
n signifie 0 ou 1
ainsi que le cas échéant leurs sels d'addition d'acide physiologiquement acceptables.

2. Composés de formule I selon la revendication 1,
où n représente 1 et R³ signifie hydrogène.

3. Composés de formule I selon l'une quelconque des revendications précédentes, où Ar² représente indolyle ou benzothiophényle.

4. Composés selon la revendication 1 de formule générale Ia, 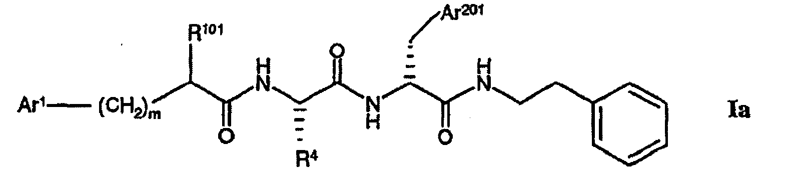 où
R¹⁰¹ signifie hydrogène ou amino,
R⁴ signifie hydrogène, C₁₋₄-alkyle ou C₁₋₄-alkylcarbonylamide,
Ar¹ signifie phényle, qui est le cas échéant monosubstitué ou disubstitué par halogène ou par C₁₋₄-alkyle ou par C₁₋₂-alkylènedioxy lié à deux atomes de carbone du cycle adjacents, pyridyle, furyle, indolyle ou tétrahydro-isoquinoléyle,
Ar²⁰¹ signifie benzothiophényle ou indolyle et
m signifie 0 ou 1
ainsi que le cas échéant leurs sels d'addition d'acide physiologiquement acceptables.

5. Composés selon la revendication 1 de formule générale Ib, 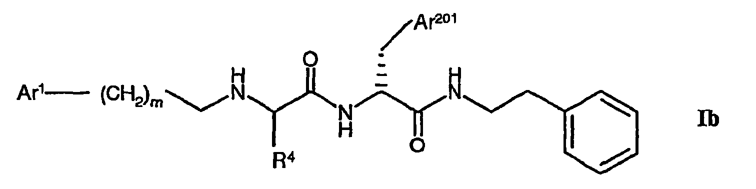 où
R⁴ signifie hydrogène, C₁₋₄-alkyle ou C₁₋₄-alkylcarbonylamide,
Ar¹ signifie phényle, qui est le cas échéant monosubstitué ou disubstitué par halogène ou par C₁₋₄-alkyle ou par C₁₋₂-alkylènedioxy lié à deux atomes de carbone du cycle adjacents, pyridyle, furyle, indolyle ou tétrahydro-isoquinoléyle,
Ar²⁰¹ signifie benzothiophényle ou indolyle et
m signifie 0 ou 1
ainsi que le cas échéant leurs sels d'addition d'acide physiologiquement acceptables.

6. Composés selon la revendication 1 de formule générale Ic, 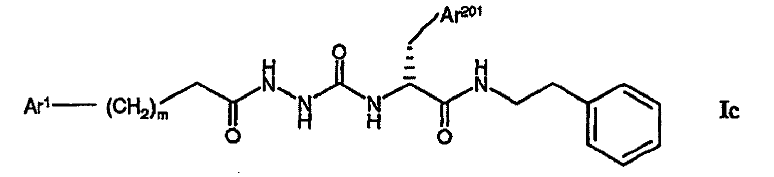 où
Ar¹ signifie phényle, qui est le cas échéant monosubstitué ou disubstitué par halogène ou par C₁₋₄-alkyle ou par C₁₋₂-alkylènedioxy lié à deux atomes de carbone du cycle adjacents, pyridyle, furyle, indolyle ou tétrahydro-isoquinoléyle,
Ar²⁰¹ signifie benzothiophényle ou indolyle et
m signifie 0 ou 1
ainsi que le cas échéant leurs sels d'addition d'acide physiologiquement acceptables.

7. Composés selon la revendication 1 de formule générale Id, 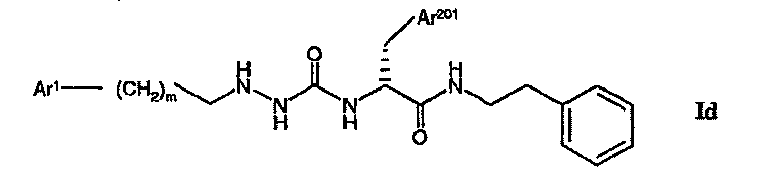 où
Ar¹ signifie phényle, qui est le cas échéant monosubstitué ou disubstitué par halogène ou par C₁₋₄-alkyle ou par C₁₋₂-alkylènedioxy lié à deux atomes de carbone du cycle adjacents, pyridyle, furyle, indolyle ou tétrahydro-isoquinoléyle,
Ar²⁰¹ signifie benzothiophényle ou indolyle et
m signifie 0 ou 1
ainsi que le cas échéant leurs sels d'addition d'acide physiologiquement acceptables.

8. Composés selon la revendication 1 de formule générale Ie, 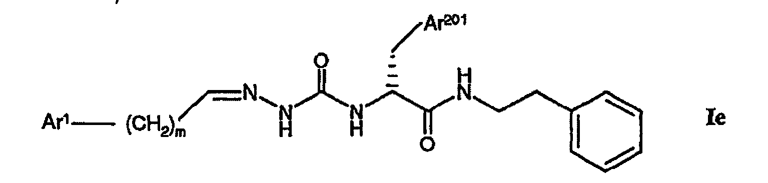 où
Ar¹ signifie phényle, qui est le cas échéant monosubstitué ou disubstitué par halogène ou par C₁₋₄-alkyle ou par C₁₋₂-alkylènedioxy lié à deux atomes de carbone du cycle adjacents, pyridyle, furyle, indolyle ou tétrahydro-isoquinoléyle,
Ar²⁰¹ signifie benzothiophényle ou indolyle et
m signifie 0 ou 1
ainsi que le cas échéant leurs sels d'addition d'acide physiologiquement acceptables.

9. Composés de formule I selon la revendication 1, destinés à une utilisation comme médicament.

10. Médicament, contenant une quantité pharmacologiquement active d'un composé de formule I selon la revendication 1 et, en plus, des adjuvants et/ou des substances support pharmaceutiques usuel (le) s.

11. Utilisation de composés de formule I selon la revendication 1 pour la préparation d'un médicament destiné au traitement et/ou la prophylaxie de l'obésité, du diabète, de l'hyperinsulinémie, des maladies cardiovasculaires, de troubles de l'appétit et/ou du syndrome X.

12. Procédé pour la préparation de composés de formule générale I, 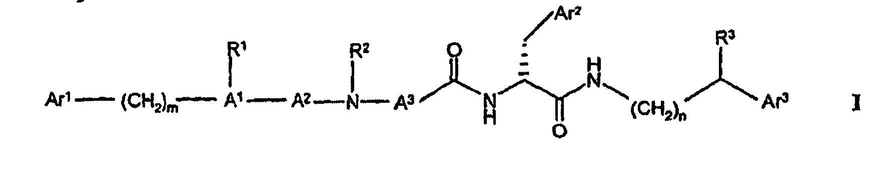 où
A¹, A², A³, R¹, R², R³, Ar¹, Ar², Ar³, m et n ont la signification mentionnée dans la revendication 1,
ainsi que le cas échéant leurs sels d'addition d'acide physiologiquement acceptables, **caractérisé en ce que**
a) pour la préparation d'un composé de formule générale Ig, 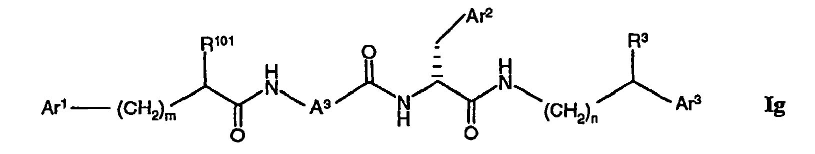 où A³, R³, Ar¹, Ar², Ar³, m et n présentent les significations ci-dessus et R¹⁰¹ signifie hydrogène
ou amino, on transforme un composé de formule générale II, 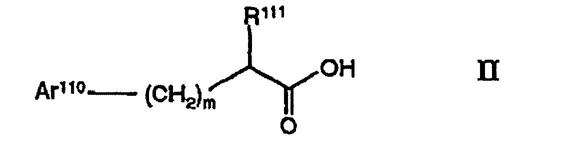 où m présente la signification ci-dessus, Ar¹¹⁰ présente la signification indiquée ci-dessus pour Ar¹, les éventuels groupes réactifs étant protégés par des groupes de protection et R¹¹¹ présente la signification indiquée ci-dessus pour R¹⁰¹, un éventuel groupe amino étant protégé par un groupe de protection, avec un composé de formule générale III, 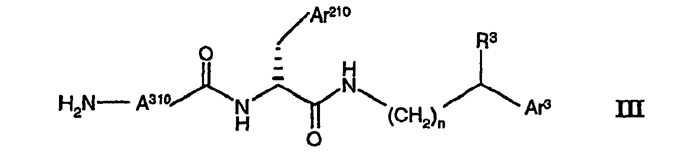 où R³, Ar³ et n présentent les significations ci-dessus, Ar²¹⁰ présente la signification indiquée ci-dessus pour Ar², les éventuels groupes réactifs étant protégés par des groupes de protection et Ar³¹⁰ présente la signification indiquée ci-dessus pour A³, les éventuels atomes d'azote réactifs étant protégés par des groupes de protection, ou
b) pour la préparation d'un composé de formule générale Ih 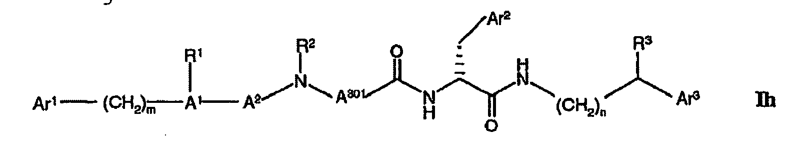 où A¹, A², R¹, R², R³, Ar¹, Ar², Ar³, m et n présentent les significations ci-dessus et A³⁰¹ présente la signification indiquée ci-dessus pour A³ à l'exception de NH, on transforme un composé de formule générale IV, 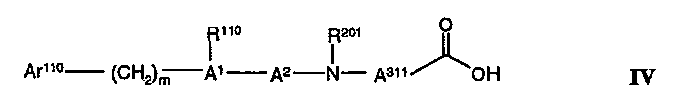 où A¹, A², Ar¹¹⁰ et m présentent les significations ci-dessus, A³¹¹ présente la signification indiquée ci-dessus pour A³⁰¹, les éventuels atomes d'azote réactifs étant protégés par des groupes de protection, R¹¹⁰ présente la signification indiquée ci-dessus pour R¹, un éventuel groupe amino étant protégé par un groupe de protection et R²⁰¹ présente la signification indiquée ci-dessus pour R² à l'exception de l'hydrogène, ou signifie un groupe de protection de la fonction amino, avec un composé de formule générale V, 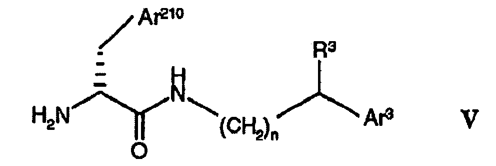 où R³, Ar²¹⁰, Ar³ et n présentent les significations ci-dessus, ou
c) pour la préparation d'un composé de formule générale Ii, 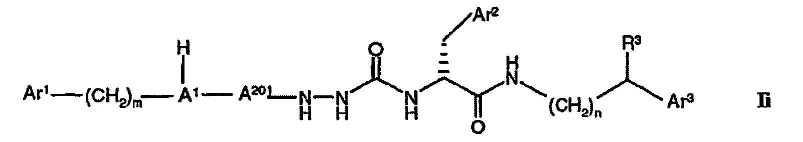 où A¹, R³, Ar¹, Ar², Ar³, m et n présentent les significations ci-dessus et A²⁰¹ présente la signification indiquée ci-dessus pour A² à l'exception de carbonyle, on transforme un composé de formule V avec un équivalent de synthèse de groupe carbonyle et avec un composé de formule générale VI, 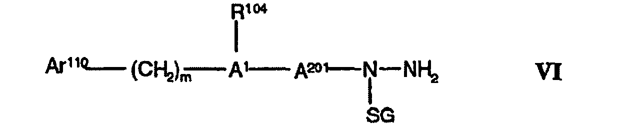 où A¹, A²⁰¹, Ar¹¹⁰ et m présentent les significations ci-dessus, R¹⁰⁴ représente hydrogène ou, pour autant que A¹ signifie azote, peut également représenter un groupe de protection de l'azote, et SG représente un groupe de protection approprié dans la chimie des peptides, ou
d) pour la préparation d'un composé de formule générale Ij 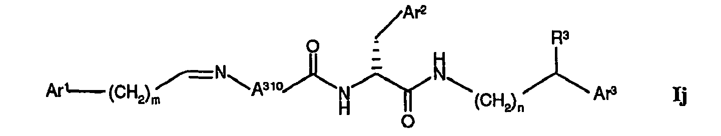
où A³¹⁰, R³, Ar¹, Ar², Ar³, m et n présentent les significations ci-dessus, on transforme un composé de formule III avec un composé de formule générale VIII, 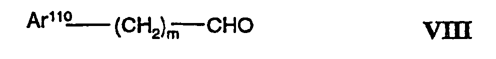 où Ar¹¹⁰ et m présentent les significations ci-dessus,
et on dissocie ensuite à nouveau les éventuels groupes de protection et on transforme un composé de formule I obtenu si souhaité en son sel d'addition d'acide ou on transforme un sel d'addition d'acide en un composé libre de formule I.
